(19) Europäisches Patentamt
European Patent Office
Office européen des brevets

(11) **EP 3 678 114 A1**

(12) **EUROPEAN PATENT APPLICATION**
published in accordance with Art. 153(4) EPC

(43) Date of publication:
**08.07.2020 Bulletin 2020/28**

(51) Int Cl.:
**G08G 1/16** (2006.01)  **A61B 5/18** (2006.01)
**B60K 28/02** (2006.01)  **B60W 40/08** (2012.01)

(21) Application number: **17923282.2**

(22) Date of filing: **31.08.2017**

(86) International application number:
**PCT/JP2017/031498**

(87) International publication number:
**WO 2019/043896 (07.03.2019 Gazette 2019/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventor: **YAMAJI, Takayuki**
**Kawasaki-shi**
**Kanagawa 211-8588 (JP)**

(74) Representative: **Hoffmann Eitle**
**Patent- und Rechtsanwälte PartmbB**
**Arabellastraße 30**
**81925 München (DE)**

(54) **DETECTION PROGRAM, DETECTION METHOD, AND DETECTION SYSTEM**

(57)    A detection device (101) acquires image information obtained by imaging a subject in a moving body (M). Furthermore, the detection device (101) acquires illuminance information indicating an illuminance in the moving body (M). The detection device (101) generates first feature information regarding a time-series change in a pupil diameter of the subject in the moving body (M) on the basis of the acquired image information. The detection device (101) generates second feature information regarding a time-series change in the illuminance in the moving body (M) on the basis of the acquired illuminance information. The detection device (101) generates reaction information regarding a reflection speed of the pupil of the subject with respect to light emitted to the subject in the moving body (M) on the basis of the generated first feature information and second feature information. The detection device (101) detects a fatigue state of the subject on the basis of the generated reaction information.

FIG. 1

**Description**

FIELD

The present invention relates to a detection program, a detection method, and a detection system.

BACKGROUND

Conventionally, it has been known that a degree of fatigue of a subject can be estimated from a pupil reflection speed that indicates how fast the pupil contracts when the subject is irradiated with visible light.

As a related art, there is a mental fatigue detection device that includes a light emitting unit that emits blinking visible light, an illumination unit that emits infrared rays, an imaging unit that images an infrared image, and a control unit. In the detection device, the light emitting unit presents the visible light of which a blinking frequency changes monotonously or in a stepwise manner to the subject. Then, the control unit obtains time-series data of a pupil diameter from a frame image included in a video including eyes of the subject imaged by the imaging unit and determines whether or not the subject is in a mental fatigue state from a change in the pupil diameter. Furthermore, there is a drowsiness detection device that images a face of the subject, detects an area of the pupil from the imaged face image, and compares the detected pupil area with a reference value so as to detect drowsiness of the subject.

CITATION LIST

PATENT DOCUMENT

Patent Document 1: Japanese Laid-open Patent Publication No. 2008-301841
Patent Document 2: Japanese Laid-open Patent Publication No. 2008-246013

SUMMARY

[TECHNICAL PROBLEM]

However, according to the related art, it is difficult to detect a fatigue state of a subject in a moving body such as a motor vehicle, a railway, or the like. For example, with the related art, it is necessary to prepare a light source that emits visible light that regularly blinks in order to determine the fatigue state from the speed of the pupil reflection of the subject. Furthermore, if a subject who is driving is continuously irradiated with blinking visible light, a load is applied to the subject, and there is a possibility that the light prevents safety driving. Therefore, this is not practical.

According to one aspect, an object of the present invention is to detect a fatigue state of a subject in a moving body.

[SOLUTION TO PROBLEM]

According to one embodiment, a detection program is provided that acquires image information obtained by imaging a subject in a moving body, acquires illuminance information indicating an illuminance in the moving body, generates first feature information regarding a time-series change in a pupil diameter of the subject on the basis of the acquired image information, generates second feature information regarding a time-series change in the illuminance in the moving body on the basis of the acquired illuminance information, generates reaction information regarding a reflection speed of a pupil of the subject with respect to light irradiated on the subject on the basis of the generated first and second feature information, and detects a fatigue state of the subject on the basis of the generated reaction information.

Furthermore, according to one embodiment, a detection program is provided that specifies a first timing when the moving body enters a tunnel and a second timing when the moving body moves out from the tunnel on the basis of information indicating a time-series change in a position of the moving body and information indicating a place where the tunnel is provided, selects measurement data regarding a subject in the moving body on the basis of the specified first and second timings, and detects a fatigue state of the subject on the basis of the selected measurement data.

[ADVANTAGEOUS EFFECTS OF INVENTION]

According to one aspect of the present invention, it is possible to detect a fatigue state of a subject in a moving body.

BRIEF DESCRIPTION OF DRAWINGS

FIG. 1 is an explanatory diagram illustrating an example of a detection method according to a first embodiment.
FIG. 2 is an explanatory diagram illustrating an example of a detection method according to a second embodiment.
FIG. 3 is an explanatory diagram illustrating an exemplary system configuration of a detection system 300 according to a third embodiment.
FIG. 4 is a block diagram illustrating an exemplary hardware configuration of a detection device 101.
FIG. 5 is a block diagram illustrating an exemplary hardware configuration of a terminal device 301.
FIG. 6 is an explanatory diagram illustrating an example of content stored in a position DB 320.
FIG. 7 is an explanatory diagram illustrating an example of content stored in an intra-tunnel pupil diameter DB 360.
FIG. 8 is an explanatory diagram illustrating an example of content stored in an intra-tunnel illuminance DB 370.
FIG. 9 is a block diagram illustrating an exemplary functional configuration of the detection device 101.
FIG. 10 is an explanatory diagram illustrating waveforms representing time-series changes in a pupil diameter and an illuminance.
FIG. 11 is an explanatory diagram illustrating a determination example of valid data indicating the time-series change in the pupil diameter of the subject.
FIG. 12 is an explanatory diagram (part 1) illustrating a specific example of feature information.
FIG. 13 is an explanatory diagram (part 2) of the specific example of the feature information.
FIG. 14 is an explanatory diagram illustrating a calculation example of a change speed of a pupil.
FIG. 15 is an explanatory diagram illustrating a part of the waveform representing the time-series change in the pupil diameter of the subject.
FIG. 16 is an explanatory diagram (part 1) illustrating an output example of a fatigue state of the subject.
FIG. 17 is an explanatory diagram (part 2) illustrating the output example of the fatigue state of the subject.
FIG. 18 is a flowchart illustrating an example of a tunnel flag determination processing procedure of the detection device 101.
FIG. 19 is a flowchart (part 1) illustrating an example of a fatigue state detection processing procedure of the detection device 101.
FIG. 20 is a flowchart (part 2) illustrating an example of the fatigue state detection processing procedure of the detection device 101.
FIG. 21 is a flowchart illustrating an example of a specific processing procedure of reaction information generation processing.

DESCRIPTION OF EMBODIMENTS

Hereinafter, embodiments of a detection program, a detection method, and a detection system according to the present invention will be described in detail with reference to the drawings.

(First Embodiment)

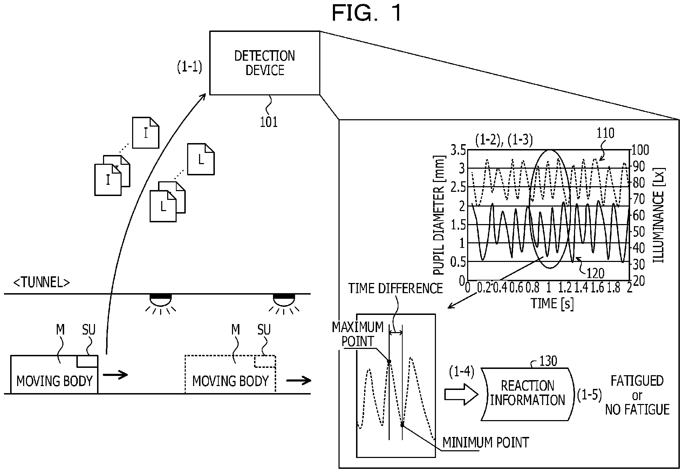

FIG. 1 is an explanatory diagram illustrating an example of a detection method according to a first embodiment. In FIG. 1, a detection device 101 is a computer that detects a fatigue state of a subject in a moving body M. The moving body M is a motor vehicle, a railway, a motorcycle, a bicycle, and the like. The motor vehicle includes an automobile, a truck, a bus, and the like. The railway includes a train, a bullet train, and the like. The subject is a person who is in the moving body M and is, for example, a driver of a motor vehicle or a railway.

As fatigue accumulates, a person tends to lose his/her judgment and attention. Therefore, it is important to grasp fatigue states of a long-distance truck driver, a railway driver, and the like and prevent an accident caused by fatigue. For example, if the fatigue state of the driver can be detected, it is possible to encourage the tired driver to take a break or switch the driver.

As a technology for estimating the fatigue state of the subject, there is a technology for detecting the fatigue state from a pupil reflection speed that indicates how fast the pupil contracts when the subject is irradiated with visible light. This is based on the fact that the pupil reflection speed is slower at the time when the subject is in a fatigued state than that at the time when the subject is not in the fatigued state, in other words, a reaction speed until the pupil contracts with respect to a visible light irradiation timing is slower.

However, if a light source that emits regularly blinking visible light is prepared to detect the fatigue state of the subject in the moving body M, installation cost and device cost are increased. Furthermore, if a subject who is driving is continuously irradiated with blinking visible light, a load is applied to the subject, and there is a possibility that the light prevents safety driving. Moreover, there is a case where sunlight that enters the moving body M from outside and the like become noise and an accurate value cannot be obtained.

Here, the tunnel is a passage that is made by digging and penetrating mountainside, underground, or the like. A plurality of illuminations (light source) is provided in the tunnel. At a time when the subject passes through the tunnel by the moving body M, an environment in the moving body M is similar to a state where light is emitted by a "blinking light source". Furthermore, there is almost no influence of natural light in the tunnel. Moreover, in many cases, the tunnel is a one-way street or a light shielding fence is provided between a lane and an oncoming traffic lane in the tunnel. There is almost no influence of light of an oncoming vehicle.

Therefore, it is concerned to detect the fatigue state of the subject from the pupil reflection of the subject when the subject is traveling in the tunnel. However, a speed of the moving body M that travels in the tunnel is not limited to be constant, and illumination installation intervals are not limited to be constant. Furthermore, there is a case where an emergency light is provided in the tunnel or an evacuation place brighter than other place is provided in the tunnel.

Therefore, the environment in the moving body M when traveling in the tunnel is not limited to be a state where light is regularly emitted. Unless the moving body M is in an environment in which the moving body M is irradiated with regular light such as pulsed light, it is difficult to accurately detect the fatigue state of the subject from the pupil reflection speed.

Moreover, there is a case where the subject looks at a vehicle-borne device, a meter, and the like during driving and does not always look forward (traveling direction). Furthermore, when the subject blinks, the pupil cannot be detected. If the pupil reflection speed at the time when the subject looks forward cannot be obtained, it is difficult to accurately detect the fatigue state of the subject.

Therefore, in the first embodiment, a detection method will be described for accurately detecting the fatigue state of the subject from the pupil reflection speed of the subject in the moving body M by using the environment in which light is regularly emitted into the moving body M that travels, as in the tunnel. Hereinafter, exemplary processing of the detection device 101 according to the first embodiment will be described.

(1-1) The detection device 101 acquires image information obtained by imaging the subject in the moving body M. The subject in the moving body M is a person whose fatigue state is detected and, for example, is a driver of the moving body M. Furthermore, the detection device 101 acquires illuminance information indicating an illuminance in the moving body M. The illuminance (light quantity) represents a degree of brightness of a surface irradiated with light. The illuminance information is information indicating an illuminance of light that has entered the moving body M, for example, by an illumination in a tunnel and the like.

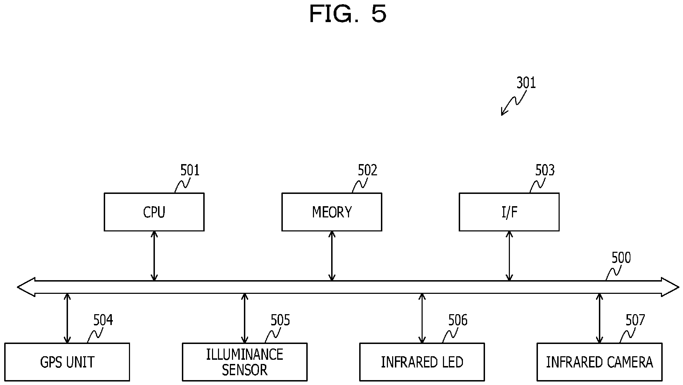

In the example in FIG. 1, a sensor unit SU is provided in the moving body M. The sensor unit SU includes an imaging device (for example, infrared camera 507 illustrated in FIG. 5 to be described later) that images the subject in the moving body M. Furthermore, the sensor unit SU includes an illuminance sensor (for example, illuminance sensor 505 illustrated in FIG. 5 to be described later) that measures the illuminance in the moving body M. The illuminance is specified, for example, from an electrical signal indicating an intensity of light detected by a light receiving element. The illuminance sensor (light receiving element) is provided at a position where the illuminance sensor can receive the light has entered the moving body M, for example, on a windshield or a dashboard. However, the illuminance may be specified, for example, from the image information obtained by imaging the inside of the moving body M.

Specifically, for example, the detection device 101 sequentially acquires image information I that is periodically imaged by the sensor unit SU. Furthermore, the detection device 101 sequentially acquires illuminance information L indicating the illuminance in the moving body M periodically measured by the sensor unit SU. An image capturing timing and an illuminance measuring timing may be the same or different from each other.

Furthermore, the detection device 101 may collectively acquire, for example, the image information I and the illuminance information L for a certain period. Note that, in a case where the sensor unit SU does not have a communication function, the detection device 101 may acquire the image information I and the illuminance information L from the sensor unit SU via another device having a communication function in the moving body M.

(1-2) The detection device 101 generates first feature information regarding a time-series change in a pupil diameter of the subject in the moving body M on the basis of the acquired image information. Here, the pupil diameter of the subject is a size (diameter) of the pupil of the subject. Specifically, for example, the detection device 101 detects the pupil of the subject from the acquired image information I and calculates the pupil diameter of the detected pupil. More specifically, for example, the detection device 101 detects an eye region of the subject from the image information I. Then, the detection device 101 performs template matching on the detected eye region so as to detect the pupil of the subject. Note that, regarding a pupil detection technology, for example, Japanese Laid-open Patent Publication No. 2017-10337 can be referred.

In the example in FIG. 1, a case where first feature information 110 is generated is assumed. The first feature information 110 is waveform information representing the time-series change in the pupil diameter (unit: mm) of the subject in the moving body M (dotted line graph in FIG. 1).

(1-3) The detection device 101 generates second feature information regarding the time-series change in the illuminance in the moving body M on the basis of the acquired illuminance information. Note that, in a case where the illuminance measured by the sensor unit SU is represented by a voltage, the detection device 101 converts the illuminance into unit of [Lx].

In the example in FIG. 1, a case where second feature information 120 is generated is assumed. The second feature information 120 is waveform information representing the time-series change in the illuminance (unit: Lx) in the moving body M (solid line graph in FIG. 1).

(1-4) The detection device 101 generates reaction information regarding a reflection speed of the pupil of the subject with respect to light emitted to the subject in the moving body M on the basis of the generated first feature information and second feature information. The reaction information is, for example, information indicating a change speed (unit: mm/s) of the pupil of the subject when the subject is irradiated with light.

Specifically, for example, the detection device 101 extracts feature points of a waveform representing the time-series change in the pupil diameter of the subject on the basis of the first feature information 110. The feature point is, for example, at least any one of a maximum point, a minimum point, or a downward change maximum point (zero cross) of the waveform. Then, the detection device 101 specifies a first valid section from a time interval between the consecutive feature points among the extracted feature points. The first valid section is a section in which the waveform of the pupil diameter having a certain width as a pulse waveform appears.

Furthermore, the detection device 101 extracts feature points of a waveform representing the time-series change in the illuminance in the moving body M on the basis of the second feature information 120. Then, the detection device 101 specifies a second valid section from a time interval between the consecutive feature points among the extracted feature points. The second valid section is a section in which the waveform of the illuminance having a certain width as a pulse waveform appears.

Next, the detection device 101 acquires valid data indicating the time-series change in the pupil diameter of the subject on the basis of the specified first valid section and second valid section. Here, the valid data is information indicating the time-series change in the pupil diameter of the subject under an environment in which the subject is irradiated with regular light such as pulsed light.

Then, the detection device 101 generates the reaction information regarding the reflection speed of the pupil of the subject on the basis of the acquired valid data. More specifically, for example, the detection device 101 calculates the reflection speed (change speed) of the pupil from a change amount and a time difference between the maximum point and the minimum point of the pupil diameter that is continuous in the valid data.

In the example in FIG. 1, a case where reaction information 130 indicating the reflection speed (unit: mm/s) of the pupil of the subject in the moving body M is generated is assumed.

(1-5) The detection device 101 detects a fatigue state of the subject on the basis of the generated reaction information. Specifically, for example, in a case where the reflection speed of the pupil indicated by the generated reaction information 130 is equal to or less than a threshold, the detection device 101 may detect that the subject is in a fatigued state. The threshold can be arbitrarily set and, for example, may be set according to the sex, the age, and the like of the subject.

As described above, according to the detection device 101 according to the first embodiment, the fatigue state of the subject in the moving body M can be accurately detected by using the environment in which the inside of the traveling moving body M is regularly irradiated with light, as in a tunnel. For example, information used for fatigue state detection can be limited to information in the section in which the subject is regularly irradiated with light by the illumination (light source) in the tunnel. Furthermore, to detect the fatigue state, it is not necessary to prepare a light source that regularly blinks. Therefore, it is possible to suppress increases in installation cost and device cost. Moreover, a load applied to the subject can be reduced as compared with a case where visible light is continuously emitted to the subject by the light source that regularly blinks.

Note that, in the example in FIG. 1, a case where the detection device 101 and the sensor unit SU are separately provided has been described as an example. However, the present invention is not limited to this. For example, the sensor unit SU may be incorporated in the detection device 101 provided in the moving body M.

(Second Embodiment)

Next, a detection device 101 according to a second embodiment will be described. Note that a part similar to the part described in the first embodiment is denoted with the same reference numeral, and description thereof will be omitted.

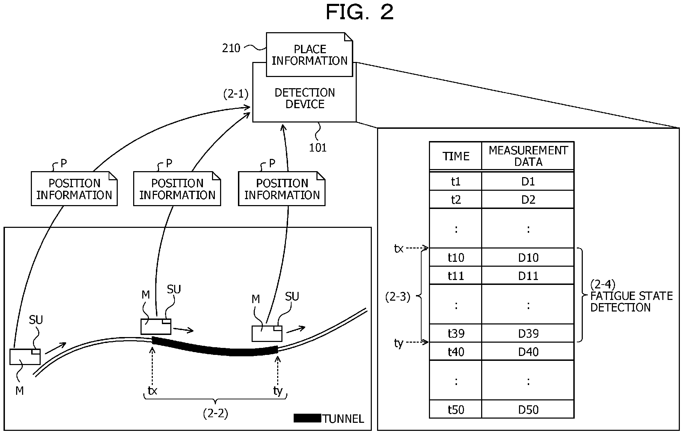

FIG. 2 is an explanatory diagram illustrating an example of a detection method according to the second embodiment. Here, since a subject travels a place other than a tunnel, if it is not distinguished whether the subject travels under an environment suitable for fatigue state detection (in tunnel) or the subject travels under an environment that is not suitable for the fatigue state detection (outside tunnel), it is difficult to accurately detect a fatigue state of the subject.

Therefore, in the second embodiment, a detection method will be described that improves detection accuracy of the fatigue state of the subject by limiting information used for the fatigue state detection to measurement data regarding the subject obtained in the tunnel (for example, image information obtained by imaging subject in moving body M). Hereinafter, exemplary processing of the detection device 101 according to the second embodiment will be described.

(2-1) The detection device 101 acquires position information indicating a position of the moving body M. The position information of the moving body M is information indicating the position of the moving body M and is, for example, information regarding the latitude and the longitude of a point where the moving body M is positioned.

In the example in FIG. 2, a sensor unit SU includes a sensor that measures the position of the moving body M. Specifically, for example, the detection device 101 sequentially acquires position information P of the moving body M that is periodically measured by the sensor unit SU.

Furthermore, the detection device 101 acquires measurement data regarding the subject in the moving body M. The subject in the moving body M is a person whose fatigue state is detected and, for example, is a driver of the moving body M. The measurement data regarding the subject is information used to detect the fatigue state of the subject and is, for example, image information obtained by imaging the subject.

Specifically, for example, the detection device 101 sequentially acquires the measurement data regarding the subject in the moving body M that is periodically measured by the sensor unit SU. In the example in FIG. 2, a case is assumed where pieces of measurement data D1 to D50 respectively measured at times t1 to t50 are acquired.

(2-2) The detection device 101 specifies a first timing when the moving body M enters the tunnel and a second timing when the moving body M moves out from the tunnel on the basis of information indicating a time-series change in the position of the moving body M and place information 210. The information indicating the time-series change in the position of the moving body M is, for example, information in which the acquired pieces of the position information of the moving body M are arranged in time series. The place information 210 is information indicating a place where the tunnel is provided.

Specifically, for example, the detection device 101 specifies the position information P in which the position of the moving body M is in the tunnel with reference to the place information 210. Then, the detection device 101 specifies the first timing when the moving body M enters the tunnel and the second timing when the moving body M moves out from the tunnel on the basis of time when the specified position information P is measured.

In the example in FIG. 2, a case is assumed where a first timing tx when the moving body M enters the tunnel and a second timing ty when the moving body M moves out from the tunnel are specified. Note that, when the moving body M is a railway (train, bullet train, and the like), there is a case where when and where the moving body M travels are managed by an operation management system. In this case, the detection device 101 may specify the first timing when the moving body M enters the tunnel and the second timing when the moving body M moves out from the tunnel by making an inquiry to the operation management system.

(2-3) The detection device 101 selects the measurement data regarding the subject in the moving body M on the basis of the specified first timing and second timing. Specifically, for example, the detection device 101 selects measurement data regarding the subject in the moving body M that is measured in a period from the first timing to the second timing.

In the example in FIG. 2, a case is assumed where the measurement data D10 to D39 that is measured in a period from the first timing tx to the second timing ty is selected from among the pieces of the measurement data D1 to D50 respectively measured at the times t1 to t50. Note that the selection of the measurement data may be performed on the measurement data that has been acquired so far, for example, in real time when the moving body M is traveling, in other words, a timing when the first timing and the second timing are specified. Furthermore, the selection of the measurement data may be performed on all the measurement data acquired during traveling after the moving body M terminates traveling.

(2-4) The detection device 101 detects the fatigue state of the subject on the basis of the selected measurement data. Specifically, for example, the detection device 101 generates feature information regarding a time-series change in a pupil diameter of the subject on the basis of the acquired measurement data D10 to D39. Next, the detection device 101 generates reaction information regarding the reflection speed of the pupil of the subject with respect to light emitted to the subject on the basis of the generated feature information. Then, the detection device 101 detects the fatigue state of the subject on the basis of the generated reaction information.

As described above, according to the detection device 101 according to the second embodiment, the information used for the fatigue state detection can be limited to the measurement data (image information) that is measured when the moving body M is traveling in the tunnel, in other words, under the environment suitable for the fatigue state detection.

As a result, for example, even when the timing when the moving body M is traveling in the tunnel and the measurement data measured when the moving body M is traveling in the tunnel are not manually designated, the fatigue state of the subject in the moving body M can be detected.

(Third Embodiment)

Next, a detection device 101 according to a third embodiment will be described. Note that a part similar to the part described in the first and second embodiments is denoted with the same reference numeral, and description thereof will be omitted. First, an exemplary system configuration of a detection system 300 including the detection device 101 according to the third embodiment will be described.

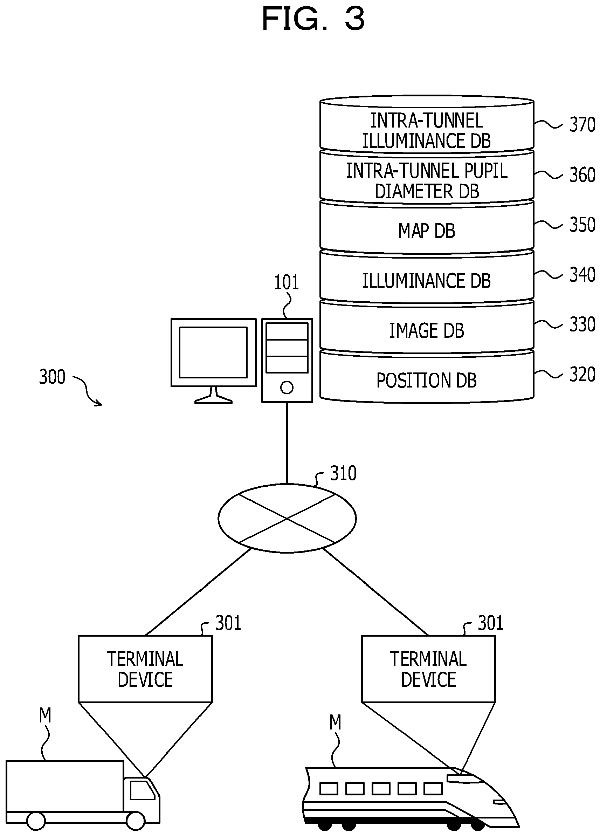

FIG. 3 is an explanatory diagram illustrating an exemplary system configuration of the detection system 300 according to the third embodiment. In FIG. 3, the detection system 300 includes the detection device 101 and a plurality of terminal devices 301 (two in example in FIG. 3). In the detection system 300, the detection device 101 and the plurality of terminal devices 301 are connected via a wired or wireless network 310. The network 310, is for example, a Local Area Network (LAN), a Wide Area Network (WAN), a moving communication network, the Internet, and the like.

The detection device 101 includes a position Database (DB) 320, an image DB 330, an illuminance DB 340, a map DB 350, an intra-tunnel pupil diameter DB 360, and an intra-tunnel illuminance DB 370 and detects a fatigue state of a subject in a moving body M. Here, the position DB 320 stores position information of the moving body M. The image DB 330 stores image information obtained by imaging the subject in the moving body M.

The illuminance DB 340 stores illuminance information indicating an illuminance in the moving body M. The map DB 350 stores map information. The map information is information in which an area where the moving body M travels is contracted at a certain rate and expressed on a plane. The intra-tunnel pupil diameter DB 360 stores information indicating a pupil diameter of the subject on the basis of the image information that obtained by performing imaging when the moving body M is traveling in a tunnel. The intra-tunnel illuminance DB 370 stores information indicating the illuminance in the moving body M when the moving body M is traveling in the tunnel.

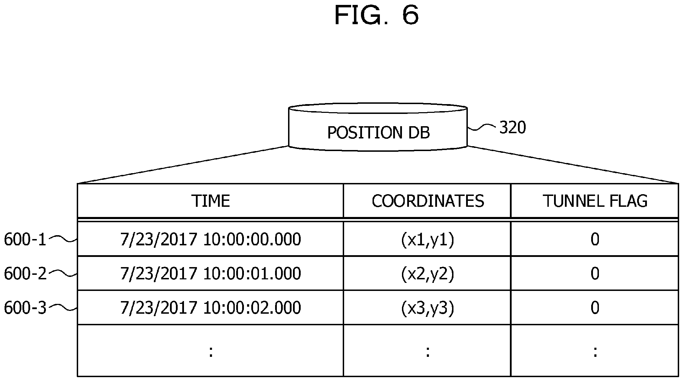

Note that content stored in the position DB 320, the intra-tunnel pupil diameter DB 360, and the intra-tunnel illuminance DB 370 will be described with reference to FIGs. 6 to 8. The detection device 101 may be, for example, a Personal Computer (PC) or a tablet PC or may be a server.

The terminal device 301 is a computer provided in the moving body M. Here, a case is assumed where the terminal device 301 can directly communicate with the detection device 101 via the network 310. However, the terminal device 301 is not limited to this. For example, the terminal device 301 may communicate with the detection device 101 via another communication device such as a smartphone.

(Exemplary Hardware Configuration of Detection Device 101)

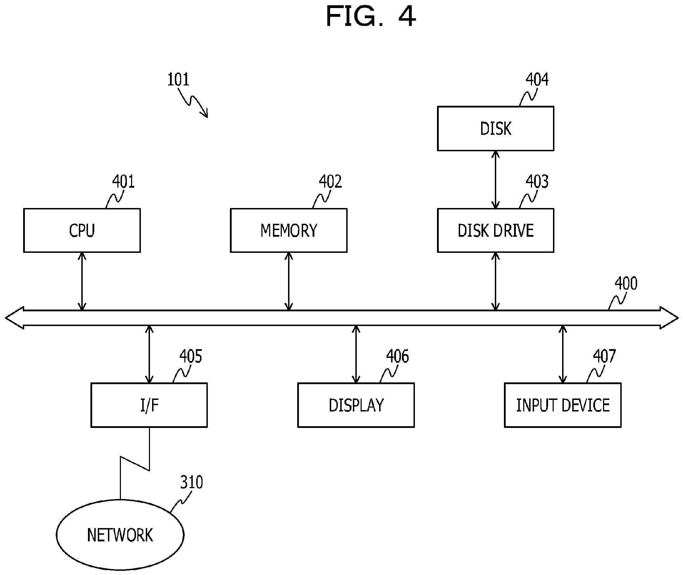

FIG. 4 is a block diagram illustrating an exemplary hardware configuration of the detection device 101. In FIG. 4, the detection device 101 includes a Central Processing Unit (CPU) 401, a memory 402, a disk drive 403, a disk 404, an Interface (I/F) 405, a display 406, and an input device 407. Furthermore, the components are connected to each other via a bus 400.

Here, the CPU 401 integrally controls the detection device 101. The memory 402 is, for example, a storage unit including a Read Only Memory (ROM), a Random Access Memory (RAM), a flash ROM, and the like. Specifically, for example, the flash ROM or the ROM stores various programs, while the RAM is used as a work area for the CPU 401. A program stored in the memory 402 is loaded into the CPU 401 to cause the CPU 401 to execute coded processing.

The disk drive 403 controls reading and writing of data with respect to the disk 404 in accordance with the control of the CPU 401. The disk 404 stores data written under the control of the disk drive 403. Examples of the disk 404 include a magnetic disk and an optical disc.

The I/F 405 is connected to the network 310 via a communication line and is connected to an external device (for example, terminal device 301 illustrated in FIG. 3) via the network 310. Then, the I/F 405 supervises an interface between the network 310 and the inside of the detection device 101 and controls input/output of data to/from the external device.

The display 406 is a display device which displays data such as a document, an image, function information, or the like, as well as a cursor, an icon, or a tool box. As the display 406, for example, a liquid crystal display, a Cathode Ray Tube (CRT), and the like can be adopted.

The input device 407 includes keys to input characters, numbers, various instructions, and the like and inputs data. The input device 407 may be a keyboard, a mouse, or the like or may be a touch-panel input pad, a numeric keypad, or the like.

Note that it is sufficient that the detection device 101 do not include, for example, the disk drive 403, the disk 404, the display 406, the input device 407, and the like from among the components described above. Furthermore, the display 406 and the input device 407 may be included in another computer connected to the detection device 101. Furthermore, detection device 101 may include, for example, a Solid State Drive (SSD), a Graphics Processing Unit (GPU), a scanner, a printer, and the like, in addition to the components described above.

(Exemplary Hardware Configuration of Terminal Device 301)

FIG. 5 is a block diagram illustrating an exemplary hardware configuration of the terminal device 301. In FIG. 5, the terminal device 301 includes a CPU 501, a memory 502, an I/F 503, a Global Positioning System (GPS) unit 504, an illuminance sensor 505, an infrared Light Emitting Diode (LED) 506, and an infrared camera 507. Furthermore, the components are connected to each other via a bus 500.

Here, the CPU 501 integrally controls the terminal device 301. The memory 502 includes, for example, a ROM, a RAM, a flash ROM, and the like. Specifically, for example, the flash ROM or the ROM stores various programs, while the RAM is used as a work area for the CPU 501. A program stored in the memory 502 is loaded into the CPU 501 to cause the CPU 501 to execute coded processing.

The I/F 503 is connected to the network 310 via a communication line and is connected to an external device (for example, detection device 101 illustrated in FIG. 3) via the network 310. Then, the I/F 503 supervises an interface between the network 310 and the inside of the terminal device 301 and controls input/output of data to/from the external device.

The GPS unit 504 outputs position information indicating a position of the terminal device 301 by receiving radio waves from a GPS satellite. The position information include, for example, coordinates (latitude and longitude) indicating the position of the terminal device 301. Here, a case where the GPS satellite is used as a positioning satellite has been described as an example. However, for example, a positioning satellite of a quasi-zenith satellite system may be used.

The illuminance sensor 505 is a sensor that measures an illuminance. The illuminance sensor 505 can adjust an interval between times when the illuminance is measured (sampling cycle) in accordance with the control of the CPU 501. The illuminance sampling cycle is, for example, one [ms]. The illuminance sensor 505 is provided at a position where the illuminance sensor 505 can receive light entered in the moving body M, for example, on a windshield or a dashboard of the moving body M. Note that the sensor unit SU illustrated in FIGs. 1 and 2 includes, for example, the illuminance sensor 505.

The infrared LED 506 is a light source that emits near infrared light. The near infrared light is light having a wavelength that is invisible to the subject. The infrared LED 506 is provided at a position where a driver of the moving body M is irradiated with near infrared light, for example, on the dashboard of the moving body M.

The infrared camera 507 is an imaging device that images the subject in the moving body M. The infrared camera 507 can adjust an interval of times when the subject is imaged (sampling cycle) in accordance with the control of the CPU 501. The sampling cycle for imaging the subject is, for example, 50 [ms]. The infrared camera 507 is provided at a position where the driver of the moving body M can be imaged, for example, on the dashboard of the moving body M. The sensor unit SU illustrated in FIGs. 1 and 2 includes, for example, the infrared LED 506 and the infrared camera 507.

Note that the terminal device 301 may include, for example, a SSD, a Hard Disk Drive (HDD), an input device, and the like, in addition to the components described above. Furthermore, the GPS unit 504, the illuminance sensor 505, the infrared LED 506, and the infrared camera 507 may be provided separately from the terminal device 301. In this case, the GPS unit 504, the illuminance sensor 505, the infrared LED 506, and the infrared camera 507 are connected to be communicable with the terminal device 301.

(Content Stored in Position DB 320)

Next, the content stored in the position DB 320 included in the detection device 101 will be described. The position DB 320 is realized by, for example, a storage device such as the memory 402, the disk 404, or the like illustrated in FIG. 4.

FIG. 6 is an explanatory diagram illustrating an example of the content stored in the position DB 320. In FIG. 6, the position DB 320 has fields of a time, coordinates, and a tunnel flag and sets information to each field so as to store the position information (for example, position information 600-1 to 600-3) as records.

Here, the time indicates a date and time when the position information of the moving body M is measured. The coordinates are the latitude and the longitude indicating the position of the moving body M. The tunnel flag indicates whether or not the position of the moving body M is in the tunnel. Here, a tunnel flag "0" indicates that the position of the moving body M is outside the tunnel. A tunnel flag "1" indicates that the position of the moving body M is in the tunnel.

(Content Stored in Intra-tunnel Pupil Diameter DB 360)

Next, the content stored in the intra-tunnel pupil diameter DB 360 included in the detection device 101 will be described. The intra-tunnel pupil diameter DB 360 is realized by, for example, a storage device such as the memory 402, the disk 404, or the like illustrated in FIG. 4.

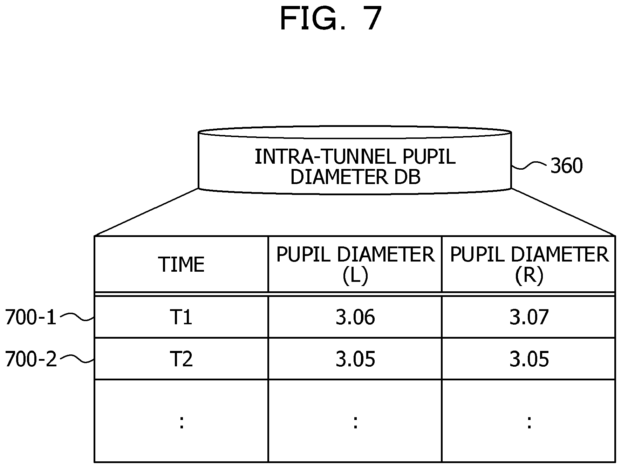

FIG. 7 is an explanatory diagram illustrating an example of the content stored in the intra-tunnel pupil diameter DB 360. In FIG. 7, the intra-tunnel pupil diameter DB 360 has fields of a time, a pupil diameter (L), and a pupil diameter (R) and sets information to each field so as to store intra-tunnel pupil diameter information (for example, intra-tunnel pupil diameter information 700-1 and 700-2) as records.

Here, the time indicates a data and time when the subject in the moving body M is imaged (for example, time to unit of millisecond). The pupil diameter (L) indicates a pupil diameter of a left eye of the subject based on the image information imaged at the corresponding time (unit: mm). The pupil diameter (R) indicates a pupil diameter of a right eye of the subject based on the image information imaged at the corresponding time (unit: mm).

(Content Stored in Intra-tunnel Illuminance DB 370)

Next, the content stored in the intra-tunnel illuminance DB 370 included in the detection device 101 will be described. The intra-tunnel illuminance DB 370 is realized by, for example, a storage device such as the memory 402, the disk 404, or the like illustrated in FIG. 4.

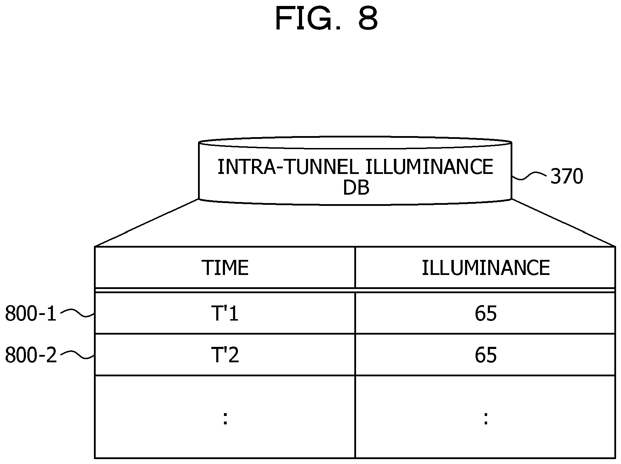

FIG. 8 is an explanatory diagram illustrating an example of the content stored in the intra-tunnel illuminance DB 370. In FIG. 8, the intra-tunnel illuminance DB 370 has fields of a time and an illuminance and sets information to each field so as to store intra-tunnel illuminance information (for example, intra-tunnel illuminance information 800-1 and 800-2) as records.

Here, the time indicates a date and time when the illuminance in the moving body M is measured (for example, time to unit of millisecond). The illuminance indicates the illuminance in the moving body M measured at the corresponding time (unit: Lx).

(Exemplary Functional Configuration of Detection Device 101)

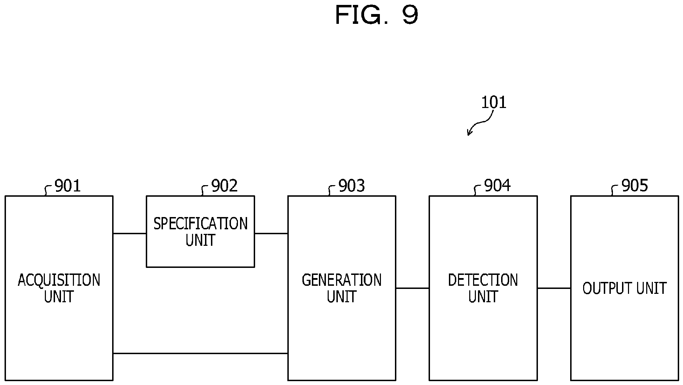

FIG. 9 is a block diagram illustrating an exemplary functional configuration of the detection device 101. In FIG. 9, the detection device 101 includes an acquisition unit 901, a specification unit 902, a generation unit 903, a detection unit 904, and an output unit 905. The acquisition unit 901 to the output unit 905 are functions to be a control unit, and specifically, for example, the functions of these parts are implemented by making the CPU 401 execute the program stored in the storage unit such as the memory 402, the disk 404, or the like illustrated in FIG. 4 or by the I/F 405. The processing result of each functional unit is stored in, for example, the storage device such as the memory 402, the disk 404, or the like.

The acquisition unit 901 acquires position information indicating the position of the moving body M. The position information of the moving body M includes, for example, a time when the position of the moving body M is measured and the latitude and the longitude indicating the position of the moving body M. Specifically, for example, the acquisition unit 901 sequentially acquires the position information of the moving body M by receiving the position information of the moving body M that is periodically measured by the GPS unit 504 of the terminal device 301 (refer to FIG. 5) from the terminal device 301.

The acquired position information of the moving body M is stored in, for example, the position DB 320 illustrated in FIG. 6. Note that the tunnel flag is "0" in an initial state.

The specification unit 902 specifies a first timing when the moving body M enters the tunnel and a second timing when the moving body M moves out from the tunnel on the basis of information indicating a time-series change in the position of the moving body M and information indicating a place where the tunnel is provided. Here, the information indicating the place where the tunnel is provided is, for example, the map information stored in the map DB 350 (refer to FIG. 3).

Specifically, for example, the specification unit 902 specifies a section (link) including a position indicated by the acquired position information of the moving body M with reference to the map DB 350. Next, the specification unit 902 determines whether or not an attribute of the specified section is "tunnel" with reference to the map DB 350. Here, in a case where the attribute of the section is "tunnel", the specification unit 902 sets "1" as the tunnel flag of the acquired position information of the moving body M. Then, the specification unit 902 specifies, with reference to the position DB 320, a time of head position information as the first timing among a plurality of continuous pieces of position information of which the tunnel flags are set to "1" in time series and a time of end position information as the second timing.

Note that, in a case where the moving body M is a railway (train, bullet train, and the like), the specification unit 902 may specify the first timing when the moving body M enters the tunnel and the second timing when the moving body M moves out from the tunnel by making an inquiry to an operation management system of the railway.

Furthermore, the acquisition unit 901 acquires image information obtained by imaging the subject in the moving body M. The image information includes, for example, a time when the subject in the moving body M is imaged. Specifically, for example, the acquisition unit 901 sequentially acquires the image information obtained by imaging the subject in the moving body M by receiving the image information that is periodically imaged by the infrared camera 507 of the terminal device 301 (refer to FIG. 5) from the terminal device 301.

The acquired image information is stored in, for example, the image DB 330 (refer to FIG. 3). Note that the acquisition unit 901 may start to acquire the image information obtained by imaging the subject in the moving body M in response to the specification of the first timing by the specification unit 902. Furthermore, the acquisition unit 901 may complete the acquisition of the image information obtained by imaging the subject in the moving body M in response to the specification of the second timing by the specification unit 902.

Furthermore, the acquisition unit 901 acquires the illuminance information indicating the illuminance in the moving body M. The illuminance information includes, for example, a time when the illuminance in the moving body M is measured. Specifically, for example, the acquisition unit 901 sequentially acquires the illuminance information indicating the illuminance in the moving body M by receiving the illuminance information indicating the illuminance that is periodically measured by the illuminance sensor 505 of the terminal device 301 (refer to FIG. 5) from the terminal device 301.

The acquired illuminance information is stored in, for example, the illuminance DB 340 (refer to FIG. 3). At this time, in a case where the illuminance indicated by the acquired illuminance information is represented by a voltage, the detection device 101 converts the illuminance into a unit of [Lx]. Note that the acquisition unit 901 may start to acquire the illuminance information indicating the illuminance in the moving body M in response to the specification of the first timing by the specification unit 902. Furthermore, the acquisition unit 901 may complete the acquisition of the illuminance information indicating the illuminance in the moving body M in response to the specification of the second timing by the specification unit 902.

The generation unit 903 generates first feature information regarding a time-series change in a pupil diameter of the subject in the moving body M on the basis of the acquired image information. Specifically, for example, the generation unit 903 selects image information imaged in a period from the first timing specified by the specification unit 902 to the second timing with reference to the image DB 330. Next, the generation unit 903 detects an eye region of the subject from the selected image information.

Then, the generation unit 903 performs template matching on the detected eye region so as to detect the pupil of the subject. Next, the generation unit 903 calculates a pupil diameter of the detected pupil. Then, the generation unit 903 generates the first feature information representing the time-series change in the pupil diameter of the subject in the moving body M by arranging the calculated pupil diameters in time series on the basis of the time of the selected image information.

Note that the generation unit 903 may calculate the pupil diameters of both eyes of the subject or may calculate the pupil diameter of either one of the left eye and the right eye of the subject. Furthermore, the generation unit 903 may calculate an average value of the pupil diameter of the left eye and the pupil diameter of the right eye as the pupil diameter of the subject.

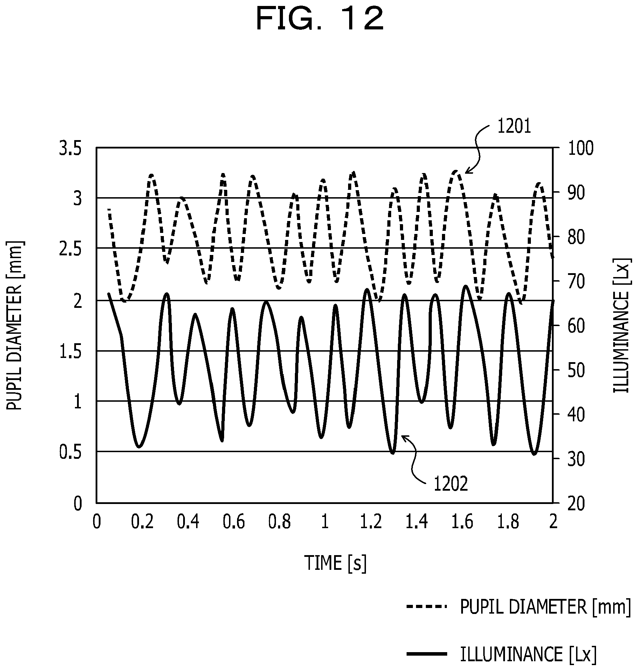

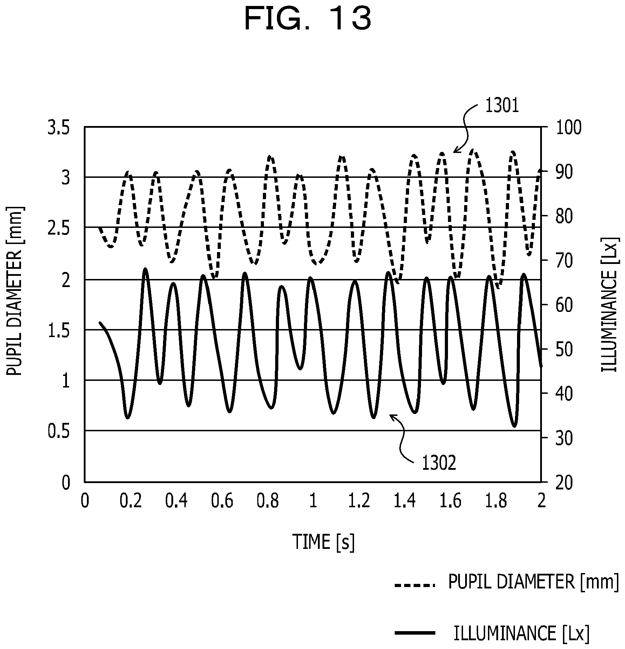

With this calculation, it is possible to generate the first feature information representing the time-series change in the pupil diameter of the subject (subject) in the moving body M that is traveling in the tunnel. The first feature information is, for example, waveform information 1201 and 1301 respectively illustrated in FIGs. 12 and 13 described later. The generated first feature information is stored in, for example, the intra-tunnel pupil diameter DB 360 illustrated in FIG. 7.

Furthermore, the generation unit 903 generates second feature information regarding a time-series change in the illuminance in the moving body M on the basis of the acquired illuminance information. Specifically, for example, the generation unit 903 selects illuminance information indicating an illuminance measured in a period from the first timing specified by the specification unit 902 and the second timing with reference to the illuminance DB 340. Then, the generation unit 903 generates the second feature information representing the time-series change in the illuminance in the moving body M by arranging the illuminances indicated by the illuminance information in time series on the basis of the time of the selected illuminance information.

With this calculation, the second feature information representing the time-series change in the illuminance in the moving body M that is traveling in the tunnel can be generated. The second feature information is, for example, waveform information 1202 and 1302 respectively illustrated in FIGs. 12 and 13 described later. The generated second feature information is stored in, for example, the intra-tunnel illuminance DB 370 illustrated in FIG. 8.

Furthermore, the generation unit 903 generates reaction information regarding a reflection speed of the pupil of the subject with respect to light emitted to the subject in the moving body M on the basis of the generated first feature information and second feature information. Specifically, for example, the generation unit 903 extracts feature points of a waveform representing the time-series change in the pupil diameter of the subject with reference to the intra-tunnel pupil diameter DB 360.

At this time, for example, the generation unit 903 may use the pupil diameter of either one of the left eye or the right eye of the subject as the pupil diameter of the subject or may use the average value of the pupil diameter of the left eye and the pupil diameter of the right eye as the pupil diameter of the subject. The feature point is, for example, at least any one of a maximum point, a minimum point, or a downward change maximum point (zero cross).

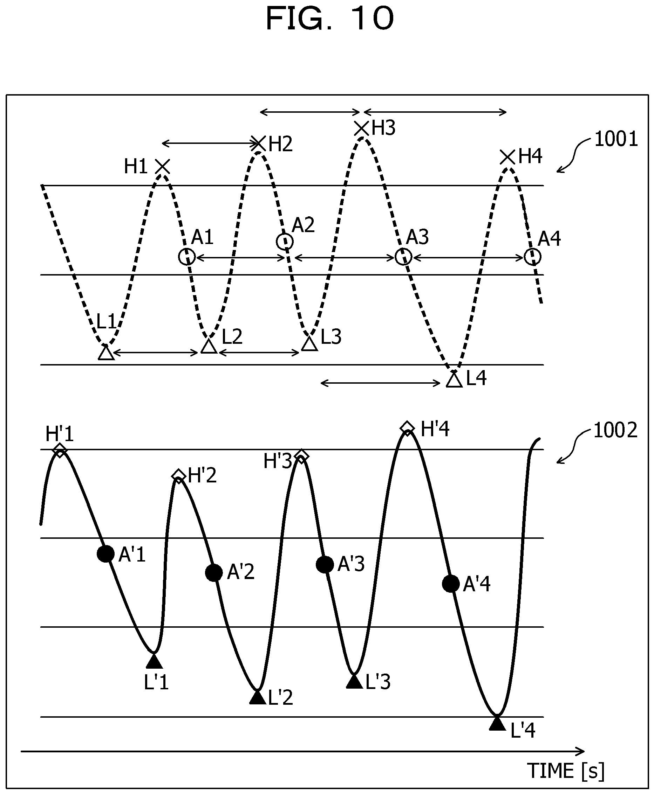

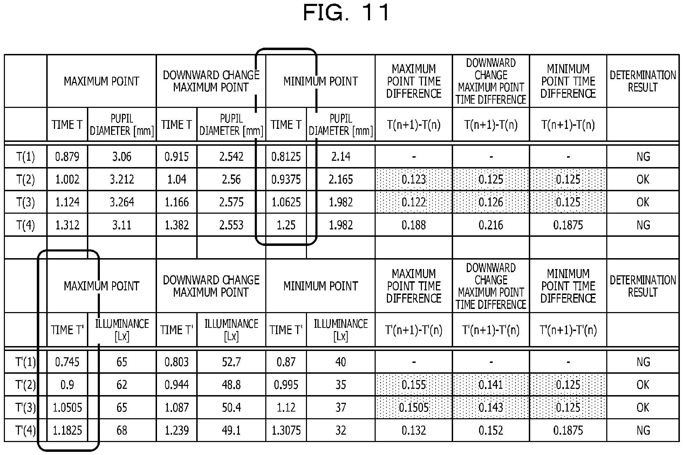

Then, the generation unit 903 specifies a first valid section from a time interval between the feature points consecutive in time series among the extracted feature points. The first valid section is a section in which the waveform of the pupil diameter having a certain width as a pulse waveform appears. A specification example of the first valid period will be described later with reference to FIGs. 10 and 11.

Furthermore, for example, the generation unit 903 extracts feature points of a waveform representing the time-series change in the illuminance in the moving body M with reference to the intra-tunnel illuminance DB 370. Then, the generation unit 903 specifies a second valid section from a time interval between the consecutive feature points among the extracted feature points. The second valid section is a section in which the waveform of the illuminance having a certain width as a pulse waveform appears. A specification example of the second valid period will be described later with reference to FIGs. 10 and 11.

Next, the generation unit 903 determines valid data indicating a time-series change in the pupil diameter of the subject on the basis of the specified first valid section and second valid section. Here, the valid data is information indicating the time-series change in the pupil diameter of the subject under an environment in which the subject is irradiated with regular light such as pulsed light. A determination example of valid data will be described later with reference to FIGs. 10 and 11.

Then, the generation unit 903 generates the reaction information regarding the reflection speed of the pupil of the subject on the basis of the determined valid data. Here, the reaction information is, for example, information indicating a change speed (unit: mm/s) of the pupil of the subject when the subject is irradiated with light. Furthermore, the reaction information may be information indicating, for example, a length of a waveform representing the time-series change in the pupil diameter of the subject when the subject is irradiated with light, that is, a so-called "stretching wave wavelength (unit: mm)".

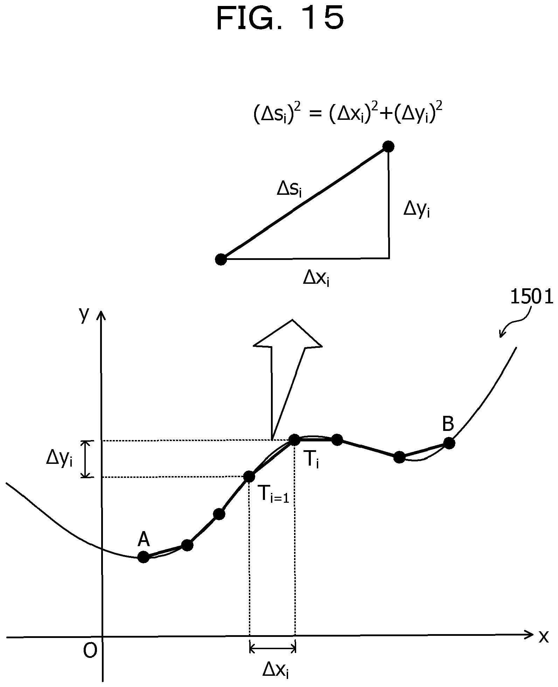

More specifically, for example, the generation unit 903 calculates the change speed of the pupil from a change amount and a time difference between the maximum point and the minimum point of the pupil diameter that is continuous in the valid data. Note that a calculation example of the change speed of the pupil will be described with reference to FIGs. 12 to 14. Furthermore, the generation unit 903 may calculate the length of the waveform from the maximum point to the minimum point of the pupil diameter that are consecutive in the valid data (stretching wave wavelength). Note that specific content of processing for calculating the stretching wave wavelength will be described with reference to FIG. 15.

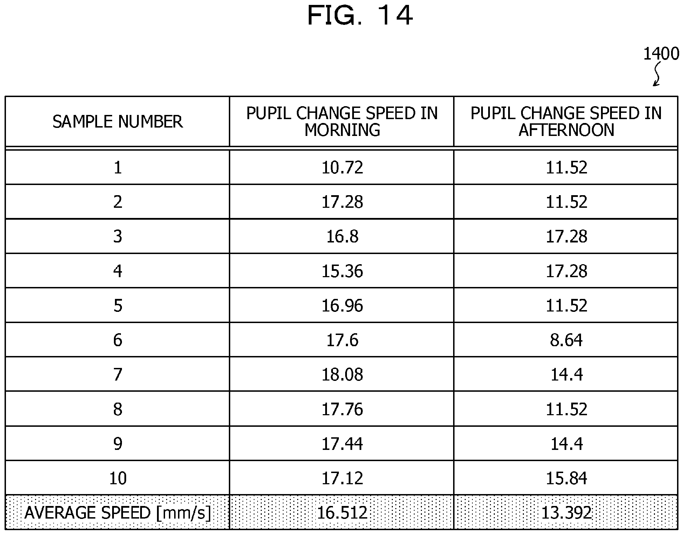

The detection unit 904 detects the fatigue state of the subject in the moving body M on the basis of the generated reaction information. Specifically, for example, in a case where the change speed of the pupil indicated by the reaction information is equal to or less than a threshold $\alpha$, the detection unit 904 detects that the subject in the moving body M is in a fatigued state. In a case where the change speed of the pupil indicated by the reaction information is larger than the threshold $\alpha$, the detection unit 904 detects that the subject in the moving body M is in a fatigue-free state. The threshold $\alpha$ can be arbitrarily set, and for example, is set according to the sex, the age, and the like of the subject. As an example, the threshold $\alpha$ is set to a value of about 14.35 [mm/s]. Note that a detection example of the fatigue state of the subject in the moving body M will be described later with reference to FIG. 14.

Furthermore, in a case where the stretching wave wavelength indicated by the reaction information is equal to or less than a threshold $\beta$, the detection unit 904 may detect that the subject in the moving body M is in the fatigued state. In a case where the stretching wave wavelength indicated by the reaction information is larger than the threshold $\beta$, the detection unit 904 detects that the subject in the moving body M is in a fatigue-free state. The threshold $\beta$ can be arbitrarily set, and for example, is set according to the sex, the age, and the like of the subject. For example, the threshold $\beta$ is set to a value of about 25.67 [mm].

The output unit 905 outputs the detected fatigue state of the subject in the moving body M. An output method by the output unit 905 is, for example, storage in a storage device such as the memory 402 or the disk 404, transmission to another computer by the I/F 405, display on the display 406, print output to a printer which is not illustrated, and the like.

Specifically, for example, the output unit 905 may output information, from which a detection time and a detection place can be specified, in association with the detected fatigue state of the subject. Here, the detection time is a time when the fatigue state of the subject is detected, and for example, is a time corresponding to the pupil diameter from which the change speed (or stretching wave wavelength) used to detect the fatigue state is calculated. The detection place is a position of the moving body M at the detection time.

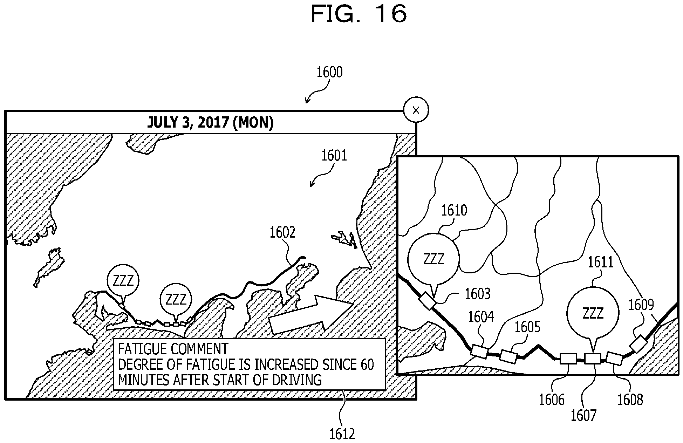

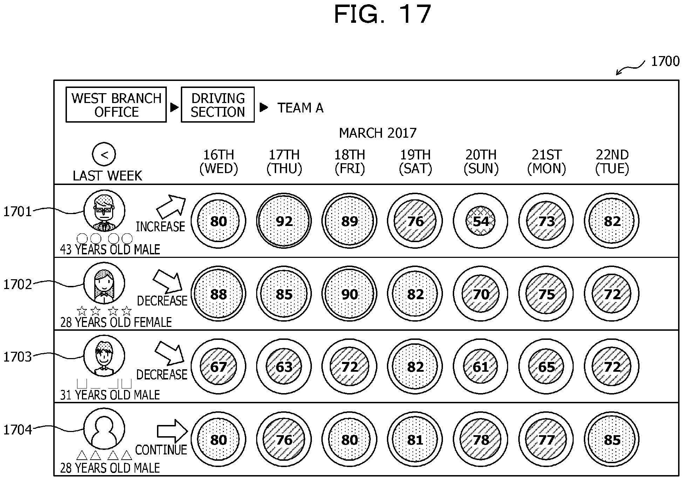

Furthermore, the output unit 905 may output information indicating a ratio of a fatigue detected section with respect to a tunnel section. Here, the fatigue detected section is a section in which the fatigued state of the subject in the moving body M is detected. For example, the tunnel section corresponds to a time length of the period from the first timing to the second timing. In a case where a plurality of periods from the first timing to the second timing exists, the tunnel section may be, for example, obtained by accumulating the time lengths of the respective periods. Furthermore, for example, it is assumed that, in the period from the first timing to the second timing, the fatigue state of the subject in the moving body M be detected on the basis of the first and the second feature information within the latest certain time (for example, latest seven seconds) for each unit time (for example, one second). In this case, the fatigue detected section is, for example, a value obtained by multiplying the number of times when the fatigued state of the subject in the moving body M is detected by the unit time (for example, one second). An output example of the fatigue state of the subject in the moving body M will be described later with reference to FIGs. 16 and 17.

Note that each functional unit of the detection device 101 may be realized by another computer different from the detection device 101 in the detection system 300, for example, the terminal device 301. Furthermore, each functional unit of the detection device 101 may be realized by a plurality of computers in the detection system 300.

(Determination Example of Valid Data)

Next, the determination example of the valid data indicating the time-series change in the pupil diameter of the subject (subject in moving body M) will be described with reference to FIGs. 10 and 11.

FIG. 10 is an explanatory diagram illustrating waveforms representing the time-series changes in the pupil diameter and the illuminance. Furthermore, FIG. 11 is an explanatory diagram illustrating the determination example of the valid data indicating the time-series change in the pupil diameter of the subject. In FIG. 10, waveform information 1001 is information representing the time-series change in the pupil diameter of the subject and corresponds to partially extracted first feature information. Furthermore, waveform information 1002 is information representing the time-series change in the illuminance in the moving body M and corresponds to partially extracted second feature information.

The generation unit 903 refers to the waveform information 1001 and extracts feature points. Here, a case is assumed where the maximum point, the minimum point, and the downward change maximum point are extracted as the feature points. In the example in FIG. 10, each of maximum points H1, H2, H3, and H4, minimum points L1, L2, L3, and L4, and downward change maximum points A1, A2, A3, and A4 is extracted.

Furthermore, the generation unit 903 refers to the waveform information 1002 and extracts feature points. In the example in FIG. 10, each of maximum points H'1, H'2, H'3, and H'4, minimum points L'1, L'2, L'3, and L'4, and downward change maximum points A'1, A'2, A'3, and A'4 is extracted.

In FIG. 11, a time T and a pupil diameter of each feature point of the waveform information 1001 are illustrated. For example, a time T of the maximum point H1 is "0.879", and a pupil diameter is "3.06". However, the year, month, day, hour, and minute of the time T are omitted, and only unit of second is illustrated. Furthermore, a time T' and an illuminance of each feature point of the waveform information 1002 are illustrated. However, the year, month, day, hour, and minute of the time T' are omitted, and only unit of second is illustrated. For example, a time T' of the maximum point H'1 is "0.745", and an illuminance is "65".

The generation unit 903 calculates a time interval between the same kind of feature points that are consecutive in time series among the extracted feature points, regarding the waveform information 1001. Note that the time interval between the feature points corresponds to a time difference between the times respectively corresponding to two feature points. In the example in FIG. 10, regarding the maximum points H1, H2, H3, and H4, the generation unit 903 calculates a time interval between the maximum points H1 and H2, a time interval between the maximum points H2 and H3, and a time interval between the maximum points H3 and H4.

Furthermore, regarding the minimum points L1, L2, L3, and L4, the generation unit 903 calculates a time interval between the minimum points L1 and L2, a time interval between the minimum points L2 and L3, and a time interval between the minimum points L3 and L4. Furthermore, regarding the downward change maximum points A1, A2, A3, and A4, the generation unit 903 calculates a time interval between the downward change maximum points A1 and A2, a time interval between the downward change maximum points A2 and A3, and a time interval between the downward change maximum points A3 and A4.

Next, the generation unit 903 determines whether or not a difference d1 between the time interval between the maximum points H1 and H2 and the time interval between the maximum points H2 and H3 is less than a threshold $\gamma$. The threshold $\gamma$ can be arbitrarily set, and for example, is set to a value of about 10 [ms]. Furthermore, the generation unit 903 determines whether or not a difference d2 between the time interval between the minimum points L1 and L2 and the time interval between the minimum points L2 and L3 is less than the threshold $\gamma$. Furthermore, the generation unit 903 determines whether or not a difference d3 between the time interval between the downward change maximum points A1 and A2 and the time interval between the downward change maximum points A2 and A3 is less than the threshold $\gamma$.

Then, in a case where all the differences d1, d2, and d3 are less than the threshold $\gamma$, the generation unit 903 specifies a period corresponding to the feature points H1 to H3, L1 to L3, and A1 to A3 as a first valid period. The period corresponding to the feature points H1 to H3, L1 to L3, and A1 to A3 is, for example, a period from the earliest time (time corresponding to minimum point L1) to the latest time (time corresponding to downward change maximum point A3) within the times respectively corresponding to the feature points H1 to H3, L1 to L3, and A1 to A3.

Thereafter, similar processing is executed as shifting the feature points one by one. Specifically, the generation unit 903 determines whether or not a difference d4 between the time interval between the maximum points H2 and H3 and the time interval between the maximum points H3 and H4 is less than the threshold $\gamma$. Furthermore, the generation unit 903 determines whether or not a difference d5 between the time interval between the minimum points L2 and L3 and the time interval between the minimum points L3 and L4 is less than the threshold $\gamma$. Furthermore, the generation unit 903 determines whether or not a difference d6 between the time interval between the downward change maximum points A2 and A3 and the time interval between the downward change maximum points A3 and A4 is less than the threshold $\gamma$.

Then, in a case where all the differences d4, d5, and d6 are less than the threshold $\gamma$, the generation unit 903 specifies a period corresponding to the feature points H2 to H4, L2 to L4, and A2 to A4 as the first valid period (determination result: OK). On the other hand, in a case where at least any one of the differences d4, d5, and d6 is equal to or more than the threshold $\gamma$, the generation unit 903 does not specify the corresponding to the feature points H2 to H4, L2 to L4, and A2 to A4 as the first valid period (determination result: NG)

In the example in FIG. 11, a period corresponding to the feature points H1 to H3, L1 to L3, and A1 to A3 and a period corresponding to the feature points H2 to H4, L2 to L4, and A2 to A4 as the first valid periods. As a result, in the waveform information 1001, a section in which the time intervals between the feature points that are consecutive in time series are substantially the same can be specified as the first valid section.

Similarly, the generation unit 903 calculates a time interval between the same kind of feature points that are consecutive in time series among the extracted feature points, regarding the waveform information 1002. In the example in FIG. 10, regarding the maximum points H'1, H'2, H'3, and H'4, the generation unit 903 calculates a time interval between the maximum points H'1 and H'2, a time interval between the maximum points H'2 and H'3, and a time interval between the maximum points H'3 and H'4.

Furthermore, regarding the minimum points L'1, L'2, L'3, and L'4, the generation unit 903 calculates a time interval between the minimum points L'1 and L'2, a time interval between the minimum points L'2 and L'3, and a time interval between the minimum points L'3 and L'4. Furthermore, regarding the downward change maximum points A'1, A'2, A'3, and A'4, the generation unit 903 calculates a time interval between the downward change maximum points A'1 and A'2, a time interval between the downward change maximum points A'2 and A'3, and a time interval between the downward change maximum points A'3 and A'4.

Next, the generation unit 903 determines whether or not a difference d'1 between the time interval between the maximum points H'1 and H'2 and the time interval between the maximum points H'2 and H'3 is less than the threshold $\gamma$. Furthermore, the generation unit 903 determines whether or not a difference d'2 between the time interval between the minimum points L'1 and L'2 and the time interval between the minimum points L'2 and L'3 is less than the threshold $\gamma$. Furthermore, the generation unit 903 determines whether or not a difference d'3 between the time interval between the downward change maximum points A'1 and A'2 and the time interval between the downward change maximum points A'2 and A'3 is less than the threshold $\gamma$. Then, in a case where all the differences d'1, d'2, and d'3 are less than the threshold $\gamma$, the generation unit 903 specifies a period corresponding to the feature points H'1 to H'3, L'1 to L'3, and A'1 to A'3 as a second valid period.

Thereafter, similar processing is executed as shifting the feature points one by one. Specifically, the generation unit 903 determines whether or not a difference d'4 between the time interval between the maximum points H'2 and H'3 and the time interval between the maximum points H'3 and H'4 is less than the threshold $\gamma$. Furthermore, the generation unit 903 determines whether or not a difference d'5 between the time interval between the minimum points L'2 and L'3 and the time interval between the minimum points L'3 and L'4 is less than the threshold $\gamma$. Furthermore, the generation unit 903 determines whether or not a difference d'6 between the time interval between the downward change maximum points A'2 and A'3 and the time interval between the downward change maximum points A'3 and A'4 is less than the threshold $\gamma$.

Then, in a case where all the differences d'4, d'5, and d'6 are less than the threshold $\gamma$, the generation unit 903 specifies a period corresponding to the feature points H'2 to H'4, L'2 to L'4, and A'2 to A'4 as the second valid period (determination result: OK). On the other hand, in a case where at least any one of the differences d'4, d'5, and d'6 is equal to or more than the threshold $\gamma$, the generation unit 903 does not specify the period corresponding to the feature points H'2 to H'4, L'2 to L'4, and A'2 to A'4 as the second valid period (determination result: NG)

In the example in FIG. 11, a period corresponding to the feature points H'1 to H'3, L'1 to L'3, and A'1 to A'3 and a period corresponding to the feature points H'2 to H'4, L'2 to L'4, and A'2 to A'4 are specified as the second valid periods. As a result, in the waveform information 1002, a section in which the time intervals between the feature points that are consecutive in time series are substantially the same can be specified as the second valid section.

Next, the generation unit 903 specifies a fatigue detection valid section on the basis of the specified first and second valid sections. Here, the fatigue detection valid section is a period in which data suitable for fatigue state detection is obtained. Specifically, for example, the generation unit 903 specifies a period in which the first valid section overlaps with the second valid section and a period in which the time T of the minimum point of the pupil diameter is positioned between the times T' of the consecutive maximum points of the illuminance as the fatigue detection valid section.

In the example in FIG. 11, the period in which the first valid section overlaps with the second valid section is a period from the time corresponding to the minimum point L1 to the time corresponding to the downward change maximum point A'4. Furthermore, in the period in which the first valid section overlaps with the second valid section, the times T of the minimum points L1, L2, and L3 of the pupil diameter exist between the times T' of the consecutive maximum points of the illuminance.

Therefore, for example, the generation unit 903 specifies a period from the time corresponding to the minimum point L1 to the time corresponding to the downward change maximum point A'4 as the fatigue detection valid section. Then, the generation unit 903 determines the valid data indicating the time-series change in the pupil diameter of the subject on the basis of the specified fatigue detection valid section. Specifically, for example, the generation unit 903 determines the intra-tunnel pupil diameter information of which the time is included in the fatigue detection valid section as the valid data with reference to the intra-tunnel pupil diameter DB 360.

As a result, the valid data indicating the time-series change in the pupil diameter of the subject under an environment in which the subject is irradiated with regular light such as pulsed light can be obtained.

Note that, in order to determine that the speed of the moving body M is constant to some extent, for example, the detection device 101 may perform frequency analysis on the illuminance measured by the illuminance sensor 505 and the pupil diameter of the subject based on the image information imaged by the infrared camera 507. Then, the detection device 101 may specify a section, in which peak values and change cycles of the illuminance and the pupil diameter are synchronized with each other, as the fatigue detection valid section.

(Calculation Example of Change Speed of Pupil)

Next, the calculation example of the change speed of the pupil of the subject (subject in moving body M) will be described with reference to FIGs. 12 to 14.

FIGs. 12 and 13 are explanatory diagrams illustrating a specific example of the feature information. In FIG. 12, waveform information 1201 and 1202 are information when the moving body M travels in a tunnel K in the morning of one day (for example, four hour after getting up). The waveform information 1201 is information representing a time-series change in a pupil diameter of a subject X in the moving body M and is an example of the first feature information. Furthermore, the waveform information 1202 is information representing a time-series change in an illuminance in the moving body M and is an example of the second feature information.

Furthermore, in FIG. 13, waveform information 1301 and 1302 are information when the moving body M travels in the tunnel K in the morning of one day (for example, four hour after getting up). The waveform information 1301 is information representing the time-series change in the pupil diameter of the subject X in the moving body M and is an example of the first feature information. Furthermore, the waveform information 1302 is information representing the time-series change in the illuminance in the moving body M and is an example of the second feature information.

Note that, in FIGs. 12 and 13, the vertical axis (left) indicates the pupil diameter (unit: mm). Furthermore, the vertical axis (right) indicates the illuminance (unit: Lx). Furthermore, the horizontal axis indicates a lapse of time (unit: s).

FIG. 14 is an explanatory diagram illustrating the calculation example of the change speed of the pupil. In FIG. 14, a table 1400 indicates a calculation result of a change speed of the pupil of the subject based on the valid data in the waveform information 1201 and 1202 (pupil change speed in the morning) and a calculation result of a change speed of the pupil of the subject based on the valid data in the waveform information 1301 and 1302 (pupil change speed in the afternoon). Here, a case is assumed where 10 samples (calculation results) are obtained in the fatigue detection valid section of about two seconds.

In this case, for example, the generation unit 903 deletes a maximum value (18.08 [mm/s]) and a minimum value (10.72 [mm/s]) one by one from the ten calculation results (pupil change speed in the morning). With this operation, an outlier can be excluded. Then, the generation unit 903 calculates an average speed of the eight calculation results (pupil change speed in the morning) obtained by deleting the maximum value and the minimum value as the change speed of the pupil. Here, the change speed of the pupil of the subject in the morning is "about 16.5 [mm/s]".

Furthermore, for example, the generation unit 903 deletes one maximum value (17.28 [mm/s]) and one minimum value (8.64 [mm/s]) from the ten calculation results (pupil change speed in the afternoon). With this operation, an outlier can be excluded. Then, the generation unit 903 calculates an average speed of the eight calculation results (pupil change speed in the afternoon) obtained by deleting the maximum value and the minimum value as the change speed of the pupil. Here, the change speed of the pupil of the subject in the afternoon is "about 13.3 [mm/s]".

Here, it is assumed that the threshold $\alpha$ be "$\alpha$ = 14.35 [mm/s]". In this case, the change speed of the pupil of the subject in the morning (16.5 [mm/s]) is larger than the threshold $\alpha$. Therefore, the detection unit 904 detects that the subject is in a fatigue-free state. In contrast, the change speed of the pupil of the subject in the afternoon (13.3 [mm/s]) is equal to or less than the threshold $\alpha$. Therefore, the detection unit 904 detects that the subject is in a fatigued state.

(Specific Content of Processing for Calculating Stretching Wave Wavelength)

Next, specific content of the processing for calculating the stretching wave wavelength (length of waveform) will be described with reference to FIG. 15.

FIG. 15 is an explanatory diagram illustrating a part of the waveform representing the time-series change in the pupil diameter of the subject. In FIG. 15, a waveform 1501 is displayed by extracting a part of a waveform representing the time-series change in the pupil diameter of the subject. The vertical axis (y) indicates the pupil diameter. Furthermore, the horizontal axis (x) indicates a lapse of time.

The generation unit 903 can calculate the stretching wave wavelength (length of waveform), for example, by using the following formula (1) according to the theorem of the definite integral. However, the reference t1 indicates a head time of a predetermined section in which a change amount of the pupil diameter is obtained. The tn indicates an end time of the predetermined section in which the change amount of the pupil diameter is obtained. The predetermined section can be arbitrarily set, and for example, may be a section for a certain time in the valid data (for example, section for two seconds) or may be a section from the maximum point to the minimum point.

[Expression 1]

$$\lim_{n\to\infty}\sum_{i=1}^{n}\sqrt{\left(\frac{\Delta x_i}{\Delta t_i}\right)^2+\left(\frac{\Delta y_i}{\Delta t_i}\right)^2}\,\Delta t_i = \int_{\alpha}^{\beta}\sqrt{\left(\frac{dx}{dt}\right)^2+\left(\frac{dy}{dt}\right)^2}\,dt = \int_{\alpha}^{\beta}\sqrt{\{v'(t)\}^2+\{v'(t)\}^2}\,dt \quad ...(1)$$

Accordingly, it is possible to generate the reaction information regarding the reflection speed of the pupil of the subject with respect to the light emitted to the subject from the length of the waveform (stretching wave wavelength) when the change amount of the pupil diameter is represented as a waveform.

(Output Example of Fatigue State of Subject)

Next, the output example of the fatigue state of the subject in the moving body M will be described with reference to FIGs. 16 and 17.

FIG. 16 is an explanatory diagram (part 1) illustrating the output example of the fatigue state of the subject. In FIG. 16, a fatigue detection result screen 1600 indicates the fatigue state of the subject in the moving body M detected by the detection unit 904 and is an example of a screen displayed for the subject. The fatigue detection result screen 1600 is displayed, for example, on a display of the terminal device 301 (not illustrated), a smartphone of the subject, or the like by the output unit 905. Note that, in FIG. 16, a part of the screen is extracted and enlarged (arrow part in FIG. 16).

Here, the subject in the moving body M is a driver of a railway. In the fatigue detection result screen 1600, a railway track 1602 on which the moving body M has traveled is displayed on a map 1601. An outlined rectangle on the railway track 1602 indicates a tunnel (for example, tunnels 1603 to 1609) existing on the railway track 1602. Icons 1610 and 1611 indicate that it is detected that the subject is in a fatigued state in the tunnels 1603 and 1607. A message 1612 is information to be displayed according to the detection result of the fatigue state.

According to the fatigue detection result screen 1600, it is found that it is detected that the subject is in a fatigued state in the tunnels 1603 and 1607. Furthermore, the subject can recognize from the message 1612 that the degree of fatigue has increased since 60 minutes after start of driving. From these, since the degree of fatigue of the subject is increased, for example, about 60 minutes after the start of driving, the subject can recognize that it is desirable to take a break by switching driving with another person and the like.

FIG. 17 is an explanatory diagram (part 2) illustrating the output example of the fatigue state of the subject. In FIG. 17, a fatigue detection result screen 1700 indicates a fatigue state of each of a plurality of subjects detected by the detection unit 904 and is an example of a screen displayed for an administrator. The administrator is a person who manages the fatigue states of the plurality of subjects. The subject is, for example, a driver of a long-distance truck or bus. The fatigue detection result screen 1700 is displayed on, for example, a PC, a smartphone, or the like used by the administrator by the output unit 905.

In the fatigue detection result screen 1700, degrees of fatigue per day of each of subjects 1701 to 1704 are displayed in time series. The degree of fatigue indicates a degree indicating how much the subject is tired. Here, the degree of fatigue is a value obtained by subtracting a ratio (%) of the fatigue detected section with respect to the tunnel section from 100. Therefore, a lower value of the degree of fatigue indicates that the degree indicating how much the subject is tired is higher.

According to the fatigue detection result screen 1700, the administrator can grasp the degree of fatigue per day for each of the subjects 1701 to 1704. Furthermore, the administrator can determine whether the fatigue of each of the subjects 1701 to 1704 tends to recover (increase) or tends to be accumulated (decrease) from the time-series change in the degree of fatigue of each of the subjects 1701 to 1704.

From these, the administrator can prompt the subject having accumulated fatigue (for example, subjects 1702 and 1703) to recover from the fatigue by making the subject take a break or reducing a work amount.

(Various Processing Procedures of Detection Device 101)

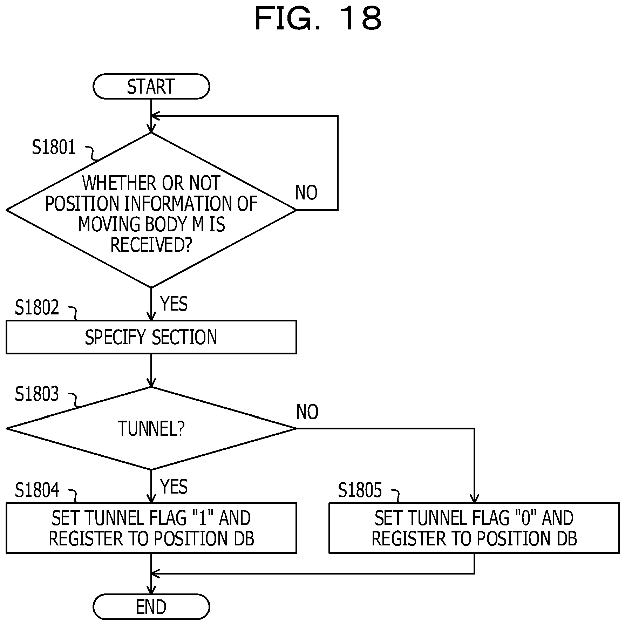

Next, various processing procedures of the detection device 101 according to the third embodiment will be described. First, a tunnel flag determination processing procedure of the detection device 101 will be described with reference to FIG. 18.

- Tunnel Flag Determination Processing Procedure

FIG. 18 is a flowchart illustrating an example of the tunnel flag determination processing procedure of the detection device 101. In the flowchart in FIG. 18, first, the detection device 101 determines whether or not the position information of the moving body M is received from the terminal device 301 (step S1801). Here, the detection device 101 waits for reception of the position information of the moving body M (step S1801: No).

Then, in a case where the position information of the moving body M is received (step S1801: Yes), the detection device 101 specifies a section including a position indicated by the acquired position information of the moving body M with reference to the map DB 350 (step S1802). Next, the detection device 101 determines whether or not an attribute of the specified section is "tunnel" with reference to the map DB 350 (step S1803).

Here, in a case where the attribute is "tunnel" (step S1803: Yes), the detection device 101 sets a tunnel flag "1" and registers the acquired position information of the moving body M to the position DB 320 (step S1804), and a series of processing according to the flowchart is terminated.

In a case where the attribute is not "tunnel" (step S1803: No), the detection device 101 sets a tunnel flag "0" and registers the acquired position information of the moving body M to the position DB 320 (step S1805), and the series of processing according to the flowchart is terminated.

With this procedure, information (tunnel flag) that can specify the first timing when the moving body M enters the tunnel and the second timing when the moving body M moves out from the tunnel can be generated.

- Fatigue State Detection Processing Procedure

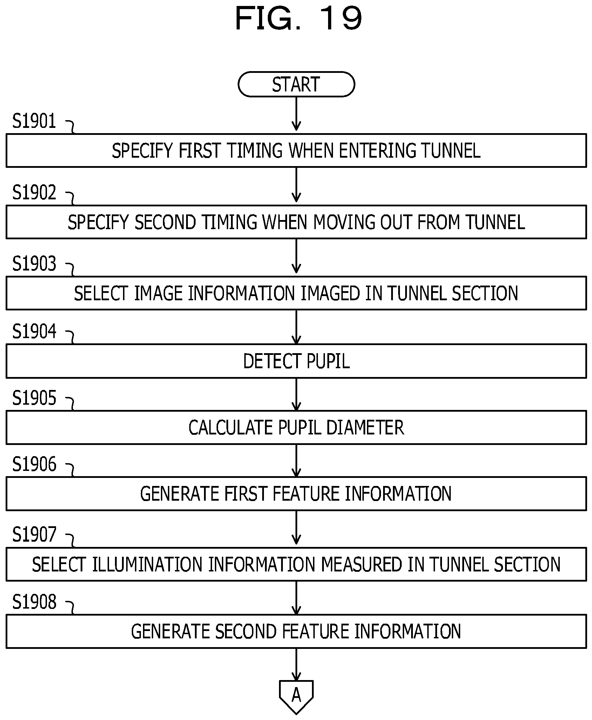

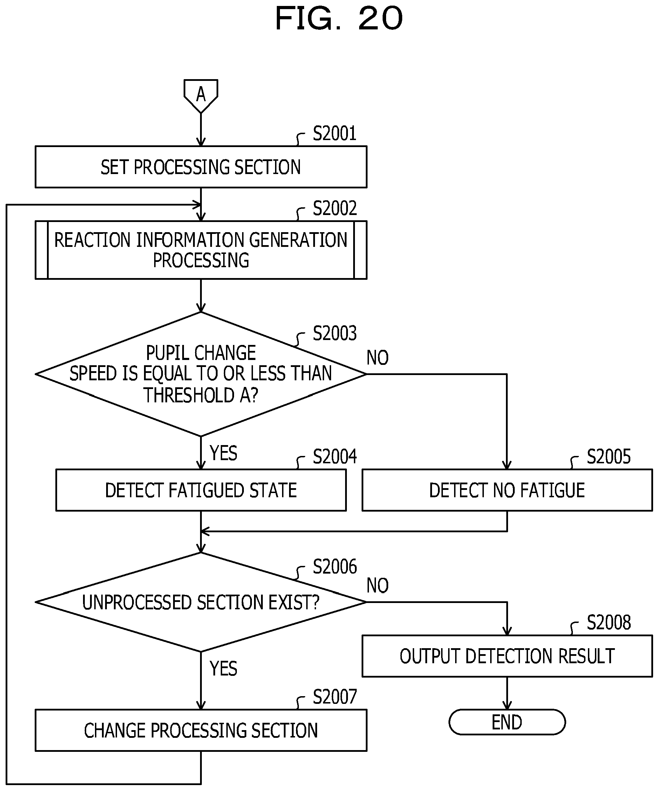

Next, a fatigue state detection processing procedure of the detection device 101 will be described with reference to FIGs. 19 and 20.

FIGs. 19 and 20 are flowcharts illustrating an example of the fatigue state detection processing procedure of the detection device 101. In the flowchart in FIG. 19, first, the detection device 101 specifies the first timing when the moving body M enters the tunnel with reference to the position DB 320 (step S1901). Next, the detection device 101 specifies the second timing when the moving body M moves out from the tunnel with reference to the position DB 320 (step S1902).

Then, the detection device 101 selects image information imaged in a tunnel section from the specified first timing to second timing with reference to the image DB 330 (step S1903). Next, the detection device 101 detects the pupil of the subject from the selected image information (step S1904) and calculates the pupil diameter of the detected pupil (step S1905).

Then, the detection device 101 generates the first feature information representing the time-series change in the pupil diameter of the subject in the moving body M by arranging the calculated pupil diameters in time series on the basis of the time of the selected image information (step S1906). The generated first feature information is stored in the intra-tunnel pupil diameter DB 360.

Next, the detection device 101 selects illuminance information indicating an illuminance measured in the tunnel section from the specified first timing to the second timing with reference to the illuminance DB 340 (step S1907).

Then, the detection device 101 generates the second feature information representing the time-series change in the illuminance in the moving body M by arranging the illuminances indicated by the illuminance information in time series on the basis of the time of the selected illuminance information (step S1908), and the procedure proceeds to step S2001 illustrated in FIG. 20. The generated second feature information is stored in the intra-tunnel illuminance DB 370.

In the flowchart in FIG. 20, first, the detection device 101 sets a processing section in the tunnel section from the first timing to the second timing (step S2001). For example, the detection device 101 sets a certain time of the tunnel section from the head of the tunnel section as the processing section. The certain time can be arbitrarily set, and for example, is set to a time about seven seconds.

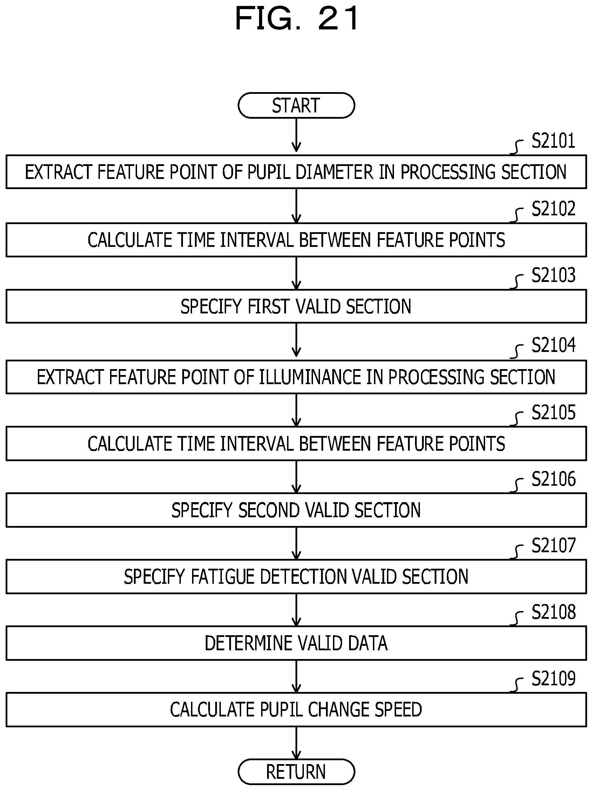

Next, the detection device 101 executes reaction information generation processing for generating the reaction information regarding the reflection speed of the pupil of the subject (step S2002). Here, a case is assumed where the reaction information indicating the change speed of the pupil of the subject when the subject is irradiated with light is generated. A specific processing procedure of the reaction information generation processing will be described later with reference to FIG. 21.

Then, the detection device 101 determines whether or not the pupil change speed indicated by the generated reaction information is equal to or less than the threshold $\alpha$ (step S2003). Here, in a case where the pupil change speed is equal to or less than the threshold $\alpha$ (step S2003: Yes), the detection device 101 detects that the subject is in a fatigued state (step S2004), and the procedure proceeds to step S2006.

In a case where the pupil change speed is larger than the threshold $\alpha$ (step S2003: No), the detection device 101 detects that the subject is in a fatigue-free state (step S2005). Then, the detection device 101 determines whether or not an unprocessed section that is not set as the processing section is included in the tunnel section (step S2006).

Here, in a case where the unprocessed section is included (step S2006: Yes), the detection device 101 changes the processing section by shifting the processing section in the tunnel section by unit time (step S2007), and the procedure returns to step S2002. Note that the unit time can be arbitrarily set, and for example, is set to a time of about one second.

In a case where the unprocessed section is not included (step S2006: No), the detection device 101 outputs a detection result in which the fatigue state of the subject is detected for each processing section (step S2008), and a series of processing according to the flowchart is terminated.

With this procedure, it is possible to detect the fatigue state of the subject by using the time-series change in the pupil diameter of the subject in the tunnel which is the environment in which the subject in the moving body M is irradiated with regular light such as pulsed light.

- Reaction Information Generation Processing Procedure

Next, a specific processing procedure of the reaction information generation processing in step S2002 illustrated in FIG. 20 will be described with reference to FIG. 21.

FIG. 21 is a flowchart illustrating an example of the specific processing procedure of the reaction information generation processing. In the flowchart in FIG. 21, first, the detection device 101 extracts feature points of a waveform representing the time-series change in the pupil diameter of the subject in the processing section set in step S2001 with reference to the intra-tunnel pupil diameter DB 360 (step S2101).

Next, the detection device 101 calculates a time interval between the consecutive feature points in time series among the extracted feature points (step S2102). Then, the detection device 101 specifies the first valid section on the basis of the calculated time interval between the feature points (step S2103).

Next, the detection device 101 extracts feature points of the waveform representing the time-series change in the illuminance in the moving body M in the processing section set in step S2001 with reference to the intra-tunnel illuminance DB 370 (step S2104). Then, the detection device 101 calculates a time interval between the consecutive feature points in time series among the extracted feature points (step S2105).

Next, the detection device 101 specifies the second valid section on the basis of the calculated time interval between the feature points (step S2106). Then, the detection device 101 specifies the fatigue detection valid section on the basis of the specified first and second valid sections (step S2107). The fatigue detection valid section is a period in which data suitable for fatigue state detection is obtained.

Next, the detection device 101 determines valid data indicating the time-series change in the pupil diameter of the subject on the basis of the specified fatigue detection valid section (step S2108). For example, the detection device 101 determines the intra-tunnel pupil diameter information of which the time is included in the fatigue detection valid section as the valid data with reference to the intra-tunnel pupil diameter DB 360.

Then, the detection device 101 calculates the pupil change speed of the subject when the subject is irradiated with light on the basis of the determined valid data (step S2109), and the procedure returns to the step that calls the reaction information generation processing. With this procedure, it is possible to generate the reaction information regarding the reflection speed of the pupil of the subject with respect to the light emitted to the subject.

As described above, according to the detection device 101 according to the embodiments, it is possible to sequentially acquire the image information obtained by imaging the subject in the moving body M and generate the first feature information regarding the time-series change in the pupil diameter of the subject on the basis of the sequentially acquired image information. Furthermore, the detection device 101 can sequentially acquire the illuminance information indicating the illuminance in the moving body M and generate the second feature information regarding the time-series change in the illuminance in the moving body M on the basis of the sequentially acquired illuminance information. Then, the detection device 101 can generate the reaction information regarding the reflection speed of the pupil of the subject with respect to the light emitted to the subject in the moving body M on the basis of the generated first and second feature information and detect the fatigue state of the subject on the basis of the generated reaction information.

With this procedure, it is possible to accurately detect the fatigue state of the subject by using the environment in which light regularly enters in the moving body M that is traveling, as in the tunnel. Furthermore, to detect the fatigue state, it is not necessary to prepare a light source that regularly blinks. Therefore, it is possible to suppress increases in installation cost and device cost. Moreover, a load applied to the subject who is driving can be reduced as compared with a case where visible light is continuously emitted to the subject by the light source that regularly blinks.

Furthermore, according to the detection device 101, it is possible to specify the first timing when the moving body M enters the tunnel and the second timing when the moving body M moves out from the tunnel on the basis of the information indicating the time-series change in the position of the moving body and the information indicating the place where the tunnel is provided. Furthermore, according to the detection device 101, it is possible to select the image information obtained by imaging the subject in the moving body M and the illuminance information indicating the illuminance in the moving body M on the basis of the specified first and second timings. Then, the detection device 101 can generate the first feature information on the basis of the selected image information and generate the second feature information on the basis of the selected illuminance information.

As a result, the information used for fatigue state detection is limited to the measurement data (image information and illuminance information) measured when the moving body M travels in the tunnel, in other words, under the environment suitable for fatigue state detection so that the fatigue state of the subject can be detected.

Furthermore, according to the detection device 101, it is possible to specify the first valid section from the time interval between the consecutive feature points among the feature points of the waveform representing the time-series change in the pupil diameter of the subject on the basis of the first feature information. The feature point is at least any one of the maximum point, the minimum point, or the downward change maximum point. As a result, it is possible to specify the section in which the time intervals between the consecutive feature points of the waveform representing the time-series change in the pupil diameter of the subject are substantially constant, in other words, the section in which the pupil diameter of the subject regularly changes as the first valid section.

Furthermore, according to the detection device 101, it is possible to specify the second valid section from the time interval between the consecutive feature points among the feature points of the waveform representing the time-series change in the illuminance in the moving body M on the basis of the second feature information. As a result, it is possible to specify the section in which the time intervals between the consecutive feature points of the waveform representing the time-series change in the illuminance in the moving body M are substantially constant, that is, a section in which regular light such as pulsed light enters the moving body M as the second valid section.

Furthermore, according to the detection device 101, it is possible to determine the valid data indicating the time-series change in the pupil diameter of the subject on the basis of the specified first and second valid sections and to generate the reaction information on the basis of the determined valid data. As a result, it is possible to detect the fatigue state of the subject from the reflection speed of the pupil of the subject that changes in response to the light that regularly enters the moving body M by the illumination (light source) in the tunnel and to improve the detection accuracy of the fatigue state of the subject.

Furthermore, according to the detection device 101, it is possible to generate the reaction information indicating the change speed of the pupil of the subject when the subject is irradiated with light on the basis of the determined valid data. Then, according to the detection device 101, in a case where the change speed of the pupil indicated by the generated reaction information is equal to or less than the threshold $\alpha$, it is possible to detect that the subject is in a fatigued state. As a result, by using the fact that the change speed of the pupil of the subject when being irradiated with light at the time when the subject is in a fatigue state is slower than that in a case where the subject is not in the fatigue state, it is possible to detect the fatigue state of the subject.

Furthermore, according to the detection device 101, it is possible to generate the reaction information indicating the length of the waveform (stretching wave wavelength) representing the time-series change in the pupil diameter of the subject when the subject is irradiated with light on the basis of the determined valid data. Then, according to the detection device 101, in a case where the stretching wave wavelength indicated by the generated reaction information is equal to or less than the threshold $\beta$, it is possible to detect that the subject is in the fatigued state. As a result, by using the fact that the change amount of the pupil diameter of the subject when the subject is irradiated with light at the time when the subject is in a fatigue state is smaller than that in a case where the subject is not in the fatigue state, it is possible to detect the fatigue state of the subject.

Furthermore, according to the detection device 101, the detected fatigue state of the subject can be output. As a result, it is possible for the subject or the administrator to grasp the fatigue state of the subject and make an appropriate response according to the fatigue state of the subject.

From these, according to the detection device 101 and the detection system 300 according to the third embodiment, it is possible to accurately detect fatigue on the basis of the pupil reflection speed of the subject in the moving body M by using the environment in the tunnel without using a light source that regularly blinks.

Note that the detection method described in this embodiment can be implemented by executing a prepared program on a computer such as a personal computer or a workstation. The detection program is recorded in a computer-readable recording medium such as a hard disk, a flexible disk, a Compact Disc (CD) -ROM, a magneto-optical (MO) disk, a digital versatile disk (DVD), and a universal serial bus (USB) memory and is executed by being read out from the recording medium by a computer. Furthermore, the detection program may be distributed via a network such as the Internet.

REFERENCE SIGNS LIST

101:detection device
110:first feature information
120:second feature information
130:reaction information
210:place information
300:detection system
301:terminal device
310:network
320:position DB
330:image DB
340:illuminance DB
350:map DB
360:intra-tunnel pupil diameter DB
370:intra-tunnel illuminance DB
400, 500:bus
401, 501:CPU
402, 502:memory
403:disk drive
404:disk
405, 503:I/F
406:display
407:input device
504:GPS unit
505:illuminance sensor
506:infrared LED
507:infrared camera
901:acquisition unit
902:specification unit
903:generation unit
904:detection unit
905:output unit
1001, 1002, 1201, 1202, 1301, 1302:waveform information
1600, 1700:fatigue detection result screen

**Claims**

1. A detection program for causing a computer to execute processing comprising:

   acquiring image information obtained by imaging a subject in a moving body;
   acquiring illuminance information that indicates an illuminance in the moving body;
   generating first feature information regarding a time-series change in a pupil diameter of the subject on the basis of the acquired image information;
   generating second feature information regarding a time-series change in the illuminance in the moving body on the basis of the acquired illuminance information;
   generating reaction information regarding a reflection speed of a pupil of the subject with respect to light emitted to the subject on the basis of the generated first and second feature information; and
   detecting a fatigue state of the subject on the basis of the generated reaction information.

2. The detection program according to claim 1, for causing the computer to execute processing comprising:

specifying a first timing when the moving body enters a tunnel and a second timing when the moving body moves out from the tunnel on the basis of information that indicates a time-series change in a position of the moving body and information that indicates a place where the tunnel is provided; and

selecting the image information obtained by imaging the subject in the moving body and the illuminance information that indicates the illuminance in the moving body on the basis of the specified first and second timings, wherein

the processing for generating the first feature information generates the first feature information on the basis of the selected image information, and

the processing for generating the second feature information generates the second feature information on the basis of the selected illuminance information.

3. The detection program according to claim 1 or 2, for causing the computer to execute processing comprising:

specifying a first valid section from a time interval between consecutive feature points of feature points of a waveform that represents the time-series change in the pupil diameter of the subject on the basis of the first feature information;

specifying a second valid section from a time interval between consecutive feature points of feature points of a waveform that represents the time-series change in the illuminance in the moving body on the basis of the second feature information;

determining valid data that indicates the time-series change in the pupil diameter of the subject on the basis of the specified first valid section and second valid section; and

generating the reaction information on the basis of the determined valid data.

4. The detection program according to claim 3, wherein
the feature point is any one of a maximum point, a minimum point, or downward change maximum point of the waveform, or combination thereof.

5. The detection program according to any one of claims 1 to 4, wherein
the reaction information indicates a change speed of the pupil of the subject when the subject is irradiated with light or a length of the waveform that represents the time-series change in the pupil diameter of the subject, and
the detecting processing
detects that the subject is in a fatigued state in a case where the change speed is equal to or less than a first threshold or the length of the waveform is equal to or less than a second threshold.

6. The detection program according to any one of claims 1 to 5, for causing the computer to execute processing comprising:
outputting the detected fatigue state of the subject.

7. The detection program according to any one of claims 1 to 6, wherein
the illuminance information is information that indicates an illuminance of light that has been output from an illuminance sensor provided in the moving body and has entered in the moving body.

8. A detection program for causing a computer to execute processing comprising:

specifying a first timing when the moving body enters a tunnel and a second timing when the moving body moves out from the tunnel on the basis of information indicating a time-series change in a position of the moving body and information that indicates a place where the tunnel is provided;

selecting measurement data regarding a subject in the moving body on the basis of the specified first and second timings; and

detecting a fatigue state of the subject on the basis of the selected measurement data.

9. A detection method for causing a computer to execute processing comprising:

acquiring image information obtained by imaging a subject in a moving body;

acquiring illuminance information that indicates an illuminance in the moving body;

generating first feature information regarding a time-series change in a pupil diameter of the subject on the basis of the acquired image information;

generating second feature information regarding a time-series change in the illuminance in the moving body on the basis of the acquired illuminance information;

generating reaction information regarding a reflection speed of a pupil of the subject with respect to light emitted to the subject on the basis of the generated first and second feature information; and

detecting a fatigue state of the subject on the basis of the generated reaction information.

10. A detection method for causing a computer to execute processing comprising:

specifying a first timing when a moving body enters a tunnel and a second timing when the moving body moves out from the tunnel on the basis of information that indicates a time-series change in a position of the moving body and information that indicates a place where the tunnel is provided;

selecting measurement data regarding a subject in the moving body on the basis of the specified first and second timings; and

detecting a fatigue state of the subject on the basis of the selected measurement data.

11. A detection system comprising:

an imaging device configured to image a subject in a moving body;

a sensor configured to measure an illuminance in the moving body; and

a detection device configured to acquire image information imaged by the imaging device, acquire illuminance information that indicates the illuminance measured by the sensor, generate first feature information regarding a time-series change in a pupil diameter of the subject on the basis of the acquired image information, generate second feature information regarding a time-series change in the illuminance in the moving body on the basis of the acquired illuminance information, generate reaction information regarding a reflection speed of a pupil of the subject with respect to light emitted to the subject on the basis of the generated first and second feature information, and detect a fatigue state of the subject on the basis of the generated reaction information.

12. A detection system comprising:

a sensor configured to measure a position of a moving body; and

a detection device configured to specify a first timing when the moving body enters a tunnel and a second timing when the moving body moves out from the tunnel on the basis of information that indicates a time-series change in a position of the moving body measured by the sensor and information that indicates a place where the tunnel is provided, select measurement data regarding a subject in the moving body on the basis of the specified first and second timings, and detect a fatigue state of the subject on the basis of the selected measurement data.

# FIG. 1

# FIG. 2

210 — PLACE INFORMATION

(2-1) DETECTION DEVICE

101

P — POSITION INFORMATION

P — POSITION INFORMATION

P — POSITION INFORMATION

SU

M

M   SU

M   SU

tx

ty

(2-2)

■ TUNNEL

| TIME | MEASUREMENT DATA |
|------|------------------|
| t1 | D1 |
| t2 | D2 |
| : | : |
| t10 | D10 |
| t11 | D11 |
| : | : |
| t39 | D39 |
| t40 | D40 |
| : | : |
| t50 | D50 |

tx ----->

ty ----->

(2-3)

(2-4) FATIGUE STATE DETECTION

# FIG. 3

INTRA-TUNNEL ILLUMINANCE DB ~ 370

INTRA-TUNNEL PUPIL DIAMETER DB ~ 360

MAP DB ~ 350

ILLUMINANCE DB ~ 340

IMAGE DB ~ 330

POSITION DB ~ 320

101

300

310

TERMINAL DEVICE 301

TERMINAL DEVICE 301

M

M

# FIG. 4

101

404
DISK

401
CPU

402
MEMORY

403
DISK DRIVE

400

405
I/F

406
DISPLAY

407
INPUT DEVICE

310
NETWORK

# FIG. 5

301

| 501 | 502 | 503 |
|-----|-----|-----|
| CPU | MEORY | I/F |

500

| 504 | 505 | 506 | 507 |
|-----|-----|-----|-----|
| GPS UNIT | ILLUMINANCE SENSOR | INFRARED LED | INFRARED CAMERA |

# FIG. 6

POSITION DB ~320

| TIME | COORDINATES | TUNNEL FLAG |
|------|-------------|-------------|
| 7/23/2017 10:00:00.000 | (x1,y1) | 0 |
| 7/23/2017 10:00:01.000 | (x2,y2) | 0 |
| 7/23/2017 10:00:02.000 | (x3,y3) | 0 |
| : | : | : |

600-1
600-2
600-3

# FIG. 7

INTRA-TUNNEL PUPIL DIAMETER DB 〜 360

| TIME | PUPIL DIAMETER (L) | PUPIL DIAMETER (R) |
|------|--------------------|--------------------|
| T1 | 3.06 | 3.07 |
| T2 | 3.05 | 3.05 |
| : | : | : |

700-1

700-2

# FIG. 8

INTRA-TUNNEL ILLUMINANCE DB ~370

| TIME | ILLUMINANCE |
|------|-------------|
| T'1 | 65 |
| T'2 | 65 |
| : | : |

800-1 (T'1 row)

800-2 (T'2 row)

# FIG. 9

# FIG. 10

# FIG. 11

| | MAXIMUM POINT | | DOWNWARD CHANGE MAXIMUM POINT | | MINIMUM POINT | | MAXIMUM POINT TIME DIFFERENCE | DOWNWARD CHANGE MAXIMUM POINT TIME DIFFERENCE | MINIMUM POINT TIME DIFFERENCE | DETERMINATION RESULT |
|---|---|---|---|---|---|---|---|---|---|---|
| | TIME T | PUPIL DIAMETER [mm] | TIME T | PUPIL DIAMETER [mm] | TIME T | PUPIL DIAMETER [mm] | T(n+1)-T(n) | T(n+1)-T(n) | T(n+1)-T(n) | |
| T(1) | 0.879 | 3.06 | 0.915 | 2.542 | 0.8125 | 2.14 | - | - | - | NG |
| T(2) | 1.002 | 3.212 | 1.04 | 2.56 | 0.9375 | 2.165 | 0.123 | 0.125 | 0.125 | OK |
| T(3) | 1.124 | 3.264 | 1.166 | 2.575 | 1.0625 | 1.982 | 0.122 | 0.126 | 0.125 | OK |
| T(4) | 1.312 | 3.11 | 1.382 | 2.553 | 1.25 | 1.982 | 0.188 | 0.216 | 0.1875 | NG |

| | MAXIMUM POINT | | DOWNWARD CHANGE MAXIMUM POINT | | MINIMUM POINT | | MAXIMUM POINT TIME DIFFERENCE | DOWNWARD CHANGE MAXIMUM POINT TIME DIFFERENCE | MINIMUM POINT TIME DIFFERENCE | DETERMINATION RESULT |
|---|---|---|---|---|---|---|---|---|---|---|
| | TIME T' | ILLUMINANCE [Lx] | TIME T' | ILLUMINANCE [Lx] | TIME T' | ILLUMINANCE [Lx] | T'(n+1)-T'(n) | T'(n+1)-T'(n) | T'(n+1)-T'(n) | |
| T'(1) | 0.745 | 65 | 0.803 | 52.7 | 0.87 | 40 | - | - | - | NG |
| T'(2) | 0.9 | 62 | 0.944 | 48.8 | 0.995 | 35 | 0.155 | 0.141 | 0.125 | OK |
| T'(3) | 1.0505 | 65 | 1.087 | 50.4 | 1.12 | 37 | 0.1505 | 0.143 | 0.125 | OK |
| T'(4) | 1.1825 | 68 | 1.239 | 49.1 | 1.3075 | 32 | 0.132 | 0.152 | 0.1875 | NG |

# FIG. 12

# FIG. 13

## FIG. 14

1400

| SAMPLE NUMBER | PUPIL CHANGE SPEED IN MORNING | PUPIL CHANGE SPEED IN AFTERNOON |
|---|---|---|
| 1 | 10.72 | 11.52 |
| 2 | 17.28 | 11.52 |
| 3 | 16.8 | 17.28 |
| 4 | 15.36 | 17.28 |
| 5 | 16.96 | 11.52 |
| 6 | 17.6 | 8.64 |
| 7 | 18.08 | 14.4 |
| 8 | 17.76 | 11.52 |
| 9 | 17.44 | 14.4 |
| 10 | 17.12 | 15.84 |
| AVERAGE SPEED [mm/s] | 16.512 | 13.392 |

# FIG. 15

$$(\Delta s_i)^2 = (\Delta x_i)^2 + (\Delta y_i)^2$$

FIG. 16

1600

JULY 3, 2017 (MON)

1601

1602

ZZZ  ZZZ

FATIGUE COMMENT
DEGREE OF FATIGUE IS INCREASED SINCE 60
MINUTES AFTER START OF DRIVING

1612

1610

ZZZ

1603
1604  1605

1611

ZZZ

1609

1606    1608
1607

# FIG. 17

1700

| WEST BRANCH OFFICE ▶ DRIVING SECTION ▶ TEAM A |
|---|

MARCH 2017

| < LAST WEEK | 16TH (WED) | 17TH (THU) | 18TH (FRI) | 19TH (SAT) | 20TH (SUN) | 21ST (MON) | 22ND (TUE) |
|---|---|---|---|---|---|---|---|
| 1701 — 43 YEARS OLD MALE — INCREASE | 80 | 92 | 89 | 76 | 54 | 73 | 82 |
| 1702 — 28 YEARS OLD FEMALE — DECREASE | 88 | 85 | 90 | 82 | 70 | 75 | 72 |
| 1703 — 31 YEARS OLD MALE — DECREASE | 67 | 63 | 72 | 82 | 61 | 65 | 72 |
| 1704 — 28 YEARS OLD MALE — CONTINUE | 80 | 76 | 80 | 81 | 78 | 77 | 85 |

# FIG. 18

START

S1801

WHETHER OR NOT
POSITION INFORMATION OF
MOVING BODY M IS
RECEIVED?

NO

YES

S1802

SPECIFY SECTION

S1803

TUNNEL?

NO

YES

S1804

SET TUNNEL FLAG "1" AND
REGISTER TO POSITION DB

S1805

SET TUNNEL FLAG "0" AND
REGISTER TO POSITION DB

END

# FIG. 19

START

S1901 — SPECIFY FIRST TIMING WHEN ENTERING TUNNEL

S1902 — SPECIFY SECOND TIMING WHEN MOVING OUT FROM TUNNEL

S1903 — SELECT IMAGE INFORMATION IMAGED IN TUNNEL SECTION

S1904 — DETECT PUPIL

S1905 — CALCULATE PUPIL DIAMETER

S1906 — GENERATE FIRST FEATURE INFORMATION

S1907 — SELECT ILLUMINATION INFORMATION MEASURED IN TUNNEL SECTION

S1908 — GENERATE SECOND FEATURE INFORMATION

A

# FIG. 20

A

SET PROCESSING SECTION ⌐ S2001

REACTION INFORMATION GENERATION PROCESSING ⌐ S2002

PUPIL CHANGE SPEED IS EQUAL TO OR LESS THAN THRESHOLD A? ⌐ S2003

NO

YES ⌐ S2004

DETECT FATIGUED STATE

DETECT NO FATIGUE ⌐ S2005

UNPROCESSED SECTION EXIST? ⌐ S2006

NO

OUTPUT DETECTION RESULT ⌐ S2008

YES

CHANGE PROCESSING SECTION ⌐ S2007

END

# FIG. 21

```
            ┌──────────┐
            │  START   │
            └────┬─────┘
                 │                                    ┌ S2101
 ┌───────────────▼──────────────────────────────────────────┐
 │ EXTRACT FEATURE POINT OF PUPIL DIAMETER IN PROCESSING SECTION │
 └───────────────┬──────────────────────────────────────────┘
                 │                                    ┌ S2102
 ┌───────────────▼──────────────────────────────────────────┐
 │      CALCULATE TIME INTERVAL BETWEEN FEATURE POINTS       │
 └───────────────┬──────────────────────────────────────────┘
                 │                                    ┌ S2103
 ┌───────────────▼──────────────────────────────────────────┐
 │             SPECIFY FIRST VALID SECTION                   │
 └───────────────┬──────────────────────────────────────────┘
                 │                                    ┌ S2104
 ┌───────────────▼──────────────────────────────────────────┐
 │ EXTRACT FEATURE POINT OF ILLUMINANCE IN PROCESSING SECTION │
 └───────────────┬──────────────────────────────────────────┘
                 │                                    ┌ S2105
 ┌───────────────▼──────────────────────────────────────────┐
 │      CALCULATE TIME INTERVAL BETWEEN FEATURE POINTS       │
 └───────────────┬──────────────────────────────────────────┘
                 │                                    ┌ S2106
 ┌───────────────▼──────────────────────────────────────────┐
 │            SPECIFY SECOND VALID SECTION                   │
 └───────────────┬──────────────────────────────────────────┘
                 │                                    ┌ S2107
 ┌───────────────▼──────────────────────────────────────────┐
 │        SPECIFY FATIGUE DETECTION VALID SECTION            │
 └───────────────┬──────────────────────────────────────────┘
                 │                                    ┌ S2108
 ┌───────────────▼──────────────────────────────────────────┐
 │                 DETERMINE VALID DATA                      │
 └───────────────┬──────────────────────────────────────────┘
                 │                                    ┌ S2109
 ┌───────────────▼──────────────────────────────────────────┐
 │            CALCULATE PUPIL CHANGE SPEED                   │
 └───────────────┬──────────────────────────────────────────┘
                 │
            ┌────▼─────┐
            │  RETURN  │
            └──────────┘
```

## INTERNATIONAL SEARCH REPORT

| | International application No. |
|---|---|
| | PCT/JP2017/031498 |

| A. CLASSIFICATION OF SUBJECT MATTER |
|---|
| *G08G1/16*(2006.01)i, *A61B5/18*(2006.01)i, *B60K28/02*(2006.01)i, *B60W40/08* *(2012.01)i* |

According to International Patent Classification (IPC) or to both national classification and IPC

| B. FIELDS SEARCHED |
|---|
| Minimum documentation searched (classification system followed by classification symbols) |
| G08G1/16, A61B5/18, B60K28/02, B60W40/08 |

| Documentation searched other than minimum documentation to the extent that such documents are included in the fields searched |
|---|
| Jitsuyo Shinan Koho          1922–1996   Jitsuyo Shinan Toroku Koho    1996–2017 Kokai Jitsuyo Shinan Koho    1971–2017   Toroku Jitsuyo Shinan Koho    1994–2017 |

| Electronic data base consulted during the international search (name of data base and, where practicable, search terms used) |
|---|
| |

### C. DOCUMENTS CONSIDERED TO BE RELEVANT

| Category* | Citation of document, with indication, where appropriate, of the relevant passages | Relevant to claim No. |
|---|---|---|
| X A | WO 2006/093074 A1  (Matsushita Electric Industrial Co., Ltd.), 08 September 2006 (08.09.2006), paragraphs [0047] to [0057] & US 2008/0117323 A1 paragraphs [0083] to [0093] & JP 6-93074 A1          & CN 101133438 A | 1,5-7,9,11 2-4,8,10,12 |
| A | JP 2008-301841 A  (National Institute of Advanced Industrial Science and Technology), 18 December 2008 (18.12.2008), paragraph [0049] & US 2010/0085539 A1 paragraph [0141] & WO 2008/149616 A1 | 3-4 |

| ☒ Further documents are listed in the continuation of Box C. | ☐ See patent family annex. |
|---|---|

\* Special categories of cited documents:
"A" document defining the general state of the art which is not considered    to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search 13 October 2017 (13.10.17) | Date of mailing of the international search report 31 October 2017 (31.10.17) |
|---|---|
| Name and mailing address of the ISA/ Japan Patent Office 3-4-3,Kasumigaseki,Chiyoda-ku, Tokyo 100-8915,Japan | Authorized officer Telephone No. |

Form PCT/ISA/210 (second sheet) (January 2015)

**INTERNATIONAL SEARCH REPORT**

International application No.

PCT/JP2017/031498

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2012-050759 A  (Hitachi, Ltd.), 15 March 2012 (15.03.2012), entire text; all drawings (Family: none) | 1-12 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008301841 A
- JP 2008246013 A
- JP 2017010337 A